# EUROPEAN PATENT APPLICATION

(11) **EP 4 071 167 A1**
(43) Date of publication of application: **12.10.2022**
(21) Application number: 20897306.5
(22) Date of filing: 03.12.2020
(51) Int. Cl.: C07K 16/00, A61K 39/00

(54) **ANTIBODY FC REGION HAVING ENHANCED FC?RIIB AFFINITY**

(30) Priority: 03.12.2019 CN 201911223227
(71) Applicant: Shanghai Jiao Tong University School of Medicine, Shanghai 200025 (CN)
(72) Inventor: LI, Fubin, Chongqing Road, Huangpu District Shanghai 200025 (CN); ZHANG, Yan, Chongqing Road, Huangpu District Shanghai 200025 (CN); ZHANG, Mi, Chongqing Road, Huangpu District Shanghai 200025 (CN); BI, Yanxia, Chongqing Road, Huangpu District Shanghai 200025 (CN); TIAN, Shihao, Chongqing Road, Huangpu District Shanghai 200025 (CN); ZHANG, Huihui, Chongqing Road, Huangpu District Shanghai 200025 (CN); LIU, Shujun, Chongqing Road, Huangpu District Shanghai 200025 (CN)
(74) Representative: IPLodge bv
(86) International application number: PCT/CN2020/133685
(87) International publication number: WO 2021/110110

(57) **Abstract**

Provided is a modified Fc region. The Fc region has at least one amino acid mutation with respect to a parent Fc region. The modified Fc region has improved affinity to FcγRIIB with respect to the affinity of the parent Fc region to FcyRIIB. Also provided is an antibody containing the modified Fc region, especially an agonistic antibody. The modified Fc region has significant use for optimizing the agonistic activity of the antibody.

Sequencing Listing

<110>  SHANGHAI JIAO TONG UNIVERSITY SCHOOL OF MEDICINE

<120>  ANTIBODY FC REGION HAVING ENHANCED BINDING AFFINITY TO FC¦ÃRIIB

<130>  P2020-2571

<150>  CN201911223227.1
<151>  2019-12-03

<160>  23

<170>  PatentIn version 3.5

<210>  1
<211>  40
<212>  DNA
<213>  Artificial sequence

<400>  1
ctaggcgccg gaattagatc taccatggag acagacagac                              40


<210>  2
<211>  36
<212>  DNA
<213>  Artificial sequence

<400>  2
gtttgttgcg ccggatccac actctcccct gttgaa                                  36


<210>  3
<211>  36
<212>  DNA
<213>  Artificial sequence

<400>  3
ttcaacaggg gagagtgtgg atccggcgca acaaac                                  36


<210>  4
<211>  36
<212>  DNA
<213>  Artificial sequence

<400>  4
gctgagcctg atgtccatgg gtccaggatt ctcttc                                  36


<210>  5
<211>  36
<212>  DNA
<213>  Artificial sequence

<400>  5
gaagagaatc ctggacccat ggacatcagg ctcagc                                  36


<210>  6
<211>  36
<212>  DNA

<213>  Artificial sequence

<400>  6
ggatggagga ggctcgagtt tacccggaga caggga                                36


<210>  7
<211>  36
<212>  DNA
<213>  Artificial sequence

<400>  7
tccctgtctc cgggtaaact cgagcctcct ccatcc                                36


<210>  8
<211>  39
<212>  DNA
<213>  Artificial sequence

<400>  8
ttcgttaacc tcgagagatc ttcacgctag agaatgagg                             39


<210>  9
<211>  699
<212>  DNA
<213>  Artificial sequence

<400>  9
atggagacag acagactcct gctatgggtg ctgctgctct gggtgccagg ctccactggt      60

gacactgtac tgacccagtc tcctgctttg gctgtgtctc caggagagag ggttaccatc     120

tcctgtaggg ccagtgacag tgtcagtaca cttatgcact ggtaccaaca gaaaccagga     180

cagcaaccca aactcctcat ctatctagca tcacacctag aatctggggt ccctgccagg     240

ttcagtggca gtgggtctgg gacagacttc accctcacca ttgatcctgt ggaggctgat     300

gacactgcaa cctattactg tcagcagagt tggaatgatc cgtggacgtt cggtggaggc     360

accaagctgg aattgaaacg aactgtggct gcaccatctg tcttcatctt cccgccatct     420

gatgagcagt tgaaatctgg aactgcctct gttgtgtgcc tgctgaataa cttctatccc     480

agagaggcca aagtacagtg gaaggtggat aacgccctcc aatcgggtaa ctcccaggag     540

agtgtcacag agcaggacag caaggacagc acctacagcc tcagcagcac cctgacgctg     600

agcaaagcag actacgagaa acacaaagtc tacgcctgcg aagtcaccca tcagggcctg     660

agctcgcccg tcacaaagag cttcaacagg ggagagtgt                            699


<210>  10
<211>  66
<212>  DNA
<213>  Artificial sequence

<400>  10
ggatccggcg caacaaactt ctctctgctg aaacaagccg gagatgtcga agagaatcct      60

ggaccc                                                                66

<210>  11
<211>  1383
<212>  DNA
<213>  Artificial sequence

<400>  11
atggacatca ggctcagctt ggttttcctt gtccttttca taaaaggtgt ccagtgtgag      60

gtgcagctgg tggagtctga tggaggctta gtgcagcctg gaaggtccct aaaactcccc     120

tgtgcagcct caggattcac tttcagtgac tattacatgg cctgggtccg ccaggctcca     180

acgaaggggc tggagtgggt cgcaagcatt agttatgatg gtagtagcac ttactatcga     240

gactccgtga agggccgatt cactatctcc agagataatg caaaaagcac cctatacctg     300

caaatggaca gtctgaggtc tgaggacacg gccacttatt actgcggaag acacagtagc     360

tactttgatt actggggcca aggagtcatg gtcacagtct cgagcgcctc caccaagggc     420

ccatcggtct tccccctggc gccctgctcc aggagcacct ccgagagcac agcggccctg     480

ggctgcctgg tcaaggacta cttccccgaa ccggtgacgg tgtcgtggaa ctcaggcgct     540

ctgaccagcg gcgtgcacac cttcccagct gtcctacagt cctcaggact ctactccctc     600

agcagcgtgg tgaccgtgcc ctccagcaac ttcggcaccc agacctacac ctgcaacgta     660

gatcacaagc ccagcaacac caaggtggac aagacagttg agcgcaaatg ttgtgtcgag     720

tgcccaccgt gcccagcacc acctgtggca ggaccgtcag tcttcctctt ccccccaaaa     780

cccaaggaca ccctcatgat ctcccggacc cctgaggtca cgtgcgtggt ggtggacgtg     840

agccacgaag accccgaggt ccagttcaac tggtacgtgg acggcgtgga ggtgcataat     900

gccaagacaa agccacggga ggagcagttc aacagcacgt tccgtgtggt cagcgtcctc     960

accgttgtgc accaggactg gctgaacggc aaggagtaca agtgcaaggt ctccaacaaa    1020

ggcctcccag cccccatcga gaaaaccatc tccaaaacca aagggcagcc ccgagaacca    1080

caggtgtaca ccctgccccc atcccgggag gagatgacca agaaccaggt cagcctgacc    1140

tgcctggtca aaggcttcta ccccagcgac atcgccgtgg agtgggagag caatgggcag    1200

ccggagaaca actacaagac cacacctccc atgctggact ccgacggctc cttcttcctc    1260

tacagcaagc tcaccgtgga caagagcagg tggcagcagg ggaacgtctt ctcatgctcc    1320

gtgatgcatg aggctctgca caaccactac acgcagaaga gcctctccct gtctccgggt    1380

aaa                                                                  1383

<210>  12
<211>  228
<212>  DNA
<213>  Artificial sequence

<400>  12
ctcgagcctc ctccatccac tgtctccaac atggcgaccg ttgctgttct ggttgtcctt      60

ggagctgcaa tagtcactgg agctgtggtg gcttttgtga tgaagatgag aaggagaaac     120

acaggtggaa aaggagggga ctatgctctg gctccaggct cccagacctc tgatctgtct     180

ctcccagatt gtaaagtgat ggttcatgac cctcattctc tagcgtga                 228

<210>  13
<211>  791
<212>  PRT
<213>  Artificial sequence

<400>  13

Met Glu Thr Asp Arg Leu Leu Leu Trp Val Leu Leu Leu Trp Val Pro
1               5                   10                  15

Gly Ser Thr Gly Asp Thr Val Leu Thr Gln Ser Pro Ala Leu Ala Val
            20                  25                  30

Ser Pro Gly Glu Arg Val Thr Ile Ser Cys Arg Ala Ser Asp Ser Val
        35                  40                  45

Ser Thr Leu Met His Trp Tyr Gln Gln Lys Pro Gly Gln Gln Pro Lys
    50                  55                  60

Leu Leu Ile Tyr Leu Ala Ser His Leu Glu Ser Gly Val Pro Ala Arg
65                  70                  75                  80

Phe Ser Gly Ser Gly Ser Gly Thr Asp Phe Thr Leu Thr Ile Asp Pro
                85                  90                  95

Val Glu Ala Asp Asp Thr Ala Thr Tyr Tyr Cys Gln Gln Ser Trp Asn
            100                 105                 110

Asp Pro Trp Thr Phe Gly Gly Gly Thr Lys Leu Glu Leu Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys Gly Ser Gly Ala Thr Asn Phe
225                 230                 235                 240

Ser Leu Leu Lys Gln Ala Gly Asp Val Glu Glu Asn Pro Gly Pro Met
                245                 250                 255

Asp Ile Arg Leu Ser Leu Val Phe Leu Val Leu Phe Ile Lys Gly Val
            260                 265                 270

Gln Cys Glu Val Gln Leu Val Glu Ser Asp Gly Gly Leu Val Gln Pro
        275                 280                 285

Gly Arg Ser Leu Lys Leu Pro Cys Ala Ala Ser Gly Phe Thr Phe Ser
    290                 295                 300

Asp Tyr Tyr Met Ala Trp Val Arg Gln Ala Pro Thr Lys Gly Leu Glu
305                 310                 315                 320

Trp Val Ala Ser Ile Ser Tyr Asp Gly Ser Ser Thr Tyr Tyr Arg Asp
                325                 330                 335

Ser Val Lys Gly Arg Phe Thr Ile Ser Arg Asp Asn Ala Lys Ser Thr
            340                 345                 350

Leu Tyr Leu Gln Met Asp Ser Leu Arg Ser Glu Asp Thr Ala Thr Tyr
        355                 360                 365

Tyr Cys Gly Arg His Ser Ser Tyr Phe Asp Tyr Trp Gly Gln Gly Val
    370                 375                 380

Met Val Thr Val Ser Ser Ala Ser Thr Lys Gly Pro Ser Val Phe Pro
385                 390                 395                 400

Leu Ala Pro Cys Ser Arg Ser Thr Ser Glu Ser Thr Ala Ala Leu Gly
                405                 410                 415

Cys Leu Val Lys Asp Tyr Phe Pro Glu Pro Val Thr Val Ser Trp Asn
            420                 425                 430

Ser Gly Ala Leu Thr Ser Gly Val His Thr Phe Pro Ala Val Leu Gln

435                 440                 445

Ser Ser Gly Leu Tyr Ser Leu Ser Ser Val Val Thr Val Pro Ser Ser
    450                 455                 460

Asn Phe Gly Thr Gln Thr Tyr Thr Cys Asn Val Asp His Lys Pro Ser
465                 470                 475                 480

Asn Thr Lys Val Asp Lys Thr Val Glu Arg Lys Cys Cys Val Glu Cys
                485                 490                 495

Pro Pro Cys Pro Ala Pro Pro Val Ala Gly Pro Ser Val Phe Leu Phe
            500                 505                 510

Pro Pro Lys Pro Lys Asp Thr Leu Met Ile Ser Arg Thr Pro Glu Val
        515                 520                 525

Thr Cys Val Val Val Asp Val Ser His Glu Asp Pro Glu Val Gln Phe
    530                 535                 540

Asn Trp Tyr Val Asp Gly Val Glu Val His Asn Ala Lys Thr Lys Pro
545                 550                 555                 560

Arg Glu Glu Gln Phe Asn Ser Thr Phe Arg Val Val Ser Val Leu Thr
                565                 570                 575

Val Val His Gln Asp Trp Leu Asn Gly Lys Glu Tyr Lys Cys Lys Val
            580                 585                 590

Ser Asn Lys Gly Leu Pro Ala Pro Ile Glu Lys Thr Ile Ser Lys Thr
        595                 600                 605

Lys Gly Gln Pro Arg Glu Pro Gln Val Tyr Thr Leu Pro Pro Ser Arg
    610                 615                 620

Glu Glu Met Thr Lys Asn Gln Val Ser Leu Thr Cys Leu Val Lys Gly
625                 630                 635                 640

Phe Tyr Pro Ser Asp Ile Ala Val Glu Trp Glu Ser Asn Gly Gln Pro
                645                 650                 655

Glu Asn Asn Tyr Lys Thr Thr Pro Pro Met Leu Asp Ser Asp Gly Ser
            660                 665                 670

Phe Phe Leu Tyr Ser Lys Leu Thr Val Asp Lys Ser Arg Trp Gln Gln
        675                 680                 685

Gly Asn Val Phe Ser Cys Ser Val Met His Glu Ala Leu His Asn His
    690                 695                 700

Tyr Thr Gln Lys Ser Leu Ser Leu Ser Pro Gly Lys Leu Glu Pro Pro
705                 710                 715                 720

Pro Ser Thr Val Ser Asn Met Ala Thr Val Ala Val Leu Val Val Leu
                725                 730                 735

Gly Ala Ala Ile Val Thr Gly Ala Val Val Ala Phe Val Met Lys Met
            740                 745                 750

Arg Arg Arg Asn Thr Gly Gly Lys Gly Gly Asp Tyr Ala Leu Ala Pro
        755                 760                 765

Gly Ser Gln Thr Ser Asp Leu Ser Leu Pro Asp Cys Lys Val Met Val
    770                 775                 780

His Asp Pro His Ser Leu Ala
785                 790

<210>  14
<211>  216
<212>  PRT
<213>  Artificial sequence

<400>  14

Ala Pro Pro Val Ala Gly Pro Ser Val Phe Leu Phe Pro Pro Lys Pro
1               5                   10                  15

Lys Asp Thr Leu Met Ile Ser Arg Thr Pro Glu Val Thr Cys Val Val
            20                  25                  30

Val Asp Val Ser His Glu Asp Pro Glu Val Gln Phe Asn Trp Tyr Val
        35                  40                  45

Asp Gly Val Glu Val His Asn Ala Lys Thr Lys Pro Arg Glu Glu Gln
    50                  55                  60

Phe Asn Ser Thr Phe Arg Val Val Ser Val Leu Thr Val Val His Gln
65                  70                  75                  80

Asp Trp Leu Asn Gly Lys Glu Tyr Lys Cys Lys Val Ser Asn Lys Gly
                85                  90                  95

Leu Pro Ala Pro Ile Glu Lys Thr Ile Ser Lys Thr Lys Gly Gln Pro
            100                 105                 110

Arg Glu Pro Gln Val Tyr Thr Leu Pro Pro Ser Arg Glu Glu Met Thr
        115                 120                 125

Lys Asn Gln Val Ser Leu Thr Cys Leu Val Lys Gly Phe Tyr Pro Ser
    130                 135                 140

Asp Ile Ala Val Glu Trp Glu Ser Asn Gly Gln Pro Glu Asn Asn Tyr
145                 150                 155                 160

Lys Thr Thr Pro Pro Met Leu Asp Ser Asp Gly Ser Phe Phe Leu Tyr
                165                 170                 175

Ser Lys Leu Thr Val Asp Lys Ser Arg Trp Gln Gln Gly Asn Val Phe
            180                 185                 190

Ser Cys Ser Val Met His Glu Ala Leu His Asn His Tyr Thr Gln Lys
        195                 200                 205

Ser Leu Ser Leu Ser Pro Gly Lys
    210                 215

<210>  15
<211>  109
<212>  PRT
<213>  Artificial sequence

<400>  15

Ala Pro Pro Val Ala Gly Pro Ser Val Phe Leu Phe Pro Pro Lys Pro
1               5                   10                  15

Lys Asp Thr Leu Met Ile Ser Arg Thr Pro Glu Val Thr Cys Val Val
            20                  25                  30

Val Asp Val Ser His Glu Asp Pro Glu Val Gln Phe Asn Trp Tyr Val
        35                  40                  45

Asp Gly Val Glu Val His Asn Ala Lys Thr Lys Pro Arg Glu Glu Gln
    50                  55                  60

Phe Asn Ser Thr Phe Arg Val Val Ser Val Leu Thr Val Val His Gln
65                  70                  75                  80

Asp Trp Leu Asn Gly Lys Glu Tyr Lys Cys Lys Val Ser Asn Lys Gly
                85                  90                  95

Leu Pro Ala Pro Ile Glu Lys Thr Ile Ser Lys Thr Lys
            100                 105

<210> 16
<211> 708
<212> DNA
<213> Artificial sequence

<400> 16
atggaaaccc cagcgcagct tctcttcctc ctgctactct ggctcccaga taccaccgga 60

gaaattgtgt tgacgcagtc tccaggcacc ctgtctttgt ctccagggga aagagccacc 120

ctctcctgca gggccagtca gagtgttagc ggcgactact tagcctggta ccagcagaaa 180

cttggccagg ctcccaggct cctcatctat ggtgcatcca tcagggccac tggcatccca 240

gacaggttca gtggcagtgg gtctgggaca gacttcactc tcaccatcag caaactggag 300

cctgaagatt ttgcagtgta ttactgtcag cagtatggta gctcaccgct cactttcggc 360

ggagggacca aggtggagat caaacgaact gtggctgcac catctgtctt catcttcccg 420

ccatctgatg agcagttgaa atctggaact gcctctgttg tgtgcctgct gaataacttc 480

tatcccagag aggccaaagt acagtggaag gtggataacg ccctccaatc gggtaactcc 540

caggagagtg tcacagagca ggacagcaag gacagcacct acagcctcag cagcaccctg 600

acgctgagca aagcagacta cgagaaacac aaagtctacg cctgcgaagt cacccatcag 660

ggcctgagct cgcccgtcac aaagagcttc aacaggggag agtgttga 708

<210> 17
<211> 1392
<212> DNA
<213> Artificial sequence

<400> 17
atgaaacacc tgtggttctt cctcctcctg gtggcagctc ccagatgggt cctgtctcag 60

gtgcagctgc aggagtcggg cccaggactg gtgaagcctt cggggaccct gtcactcacc 120

tgcgctgtct ctggtggctc catcagcagt agtatctggt ggagctgggt ccgccagccc 180

ccagggaagg ggctggaatg gattggggaa atccatcata gtgggaacac caactgcaat 240

ccgtccctca agagtcgagt caccatatca atagacaagt ccaagaacca gttctccctg 300

aaactgacct ctgtgaccgc cgcggacacg gccgtatatt attgtgcgag agcgggaact 360

ggaactacgc ttgactactg gggccaggga accctggtca ccgtctcgag cgcctccacc 420

aagggcccat cggtcttccc cctggcgccc tgctccagga gcacctccga gagcacagcg 480

gccctgggct gcctggtcaa ggactacttc cccgaaccgg tgacggtgtc gtggaactca 540

ggcgctctga ccagcggcgt gcacaccttc ccagctgtcc tacagtcctc aggactctac 600

tccctcagca gcgtggtgac cgtgccctcc agcaacttcg gcacccagac ctacacctgc 660

aacgtagatc acaagcccag caacaccaag gtggacaaga cagttgagcg caaatgttgt 720

gtcgagtgcc caccgtgccc agcaccacct gtggcaggac cgtcagtctt cctcttcccc 780

ccaaaaccca aggacaccct catgatctcc cggacccctg aggtcacgtg cgtggtggtg 840

gacgtgagcc acgaagaccc cgaggtccag ttcaactggt acgtggacgg cgtggaggtg 900

cataatgcca agacaaagcc acgggaggag cagttcaaca gcacgttccg tgtggtcagc 960

gtcctcaccg ttgtgcacca ggactggctg aacggcaagg agtacaagtg caaggtctcc 1020

aacaaaggcc tcccagcccc catcgagaaa accatctcca aaaccaaagg gcagccccga 1080

gaaccacagg tgtacaccct gcccccatcc cgggaggaga tgaccaagaa ccaggtcagc 1140

ctgacctgcc tggtcaaagg cttctacccc agcgacatcg ccgtggagtg ggagagcaat 1200

gggcagccgg agaacaacta caagaccaca cctcccatgc tggactccga cggctccttc 1260

ttcctctaca gcaagctcac cgtggacaag agcaggtggc agcaggggaa cgtcttctca 1320

tgctccgtga tgcatgaggc tctgcacaac cactacacgc agaagagcct ctccctgtct 1380

ccgggtaaat ga 1392

<210> 18
<211> 235
<212> PRT
<213> Artificial sequence

<400> 18

Met Glu Thr Pro Ala Gln Leu Leu Phe Leu Leu Leu Leu Trp Leu Pro
1               5                   10                  15

Asp Thr Thr Gly Glu Ile Val Leu Thr Gln Ser Pro Gly Thr Leu Ser
            20                  25                  30

Leu Ser Pro Gly Glu Arg Ala Thr Leu Ser Cys Arg Ala Ser Gln Ser
        35                  40                  45

Val Ser Gly Asp Tyr Leu Ala Trp Tyr Gln Gln Lys Leu Gly Gln Ala
    50                  55                  60

Pro Arg Leu Leu Ile Tyr Gly Ala Ser Ile Arg Ala Thr Gly Ile Pro
65                  70                  75                  80

Asp Arg Phe Ser Gly Ser Gly Ser Gly Thr Asp Phe Thr Leu Thr Ile
                85                  90                  95

Ser Lys Leu Glu Pro Glu Asp Phe Ala Val Tyr Tyr Cys Gln Gln Tyr
            100                 105                 110

Gly Ser Ser Pro Leu Thr Phe Gly Gly Gly Thr Lys Val Glu Ile Lys
        115                 120                 125

Arg Thr Val Ala Ala Pro Ser Val Phe Ile Phe Pro Pro Ser Asp Glu

130                 135                 140

Gln Leu Lys Ser Gly Thr Ala Ser Val Val Cys Leu Leu Asn Asn Phe
145                 150                 155                 160

Tyr Pro Arg Glu Ala Lys Val Gln Trp Lys Val Asp Asn Ala Leu Gln
                165                 170                 175

Ser Gly Asn Ser Gln Glu Ser Val Thr Glu Gln Asp Ser Lys Asp Ser
            180                 185                 190

Thr Tyr Ser Leu Ser Ser Thr Leu Thr Leu Ser Lys Ala Asp Tyr Glu
        195                 200                 205

Lys His Lys Val Tyr Ala Cys Glu Val Thr His Gln Gly Leu Ser Ser
    210                 215                 220

Pro Val Thr Lys Ser Phe Asn Arg Gly Glu Cys
225                 230                 235

<210>  19
<211>  463
<212>  PRT
<213>  Artificial sequence

<400>  19

Met Lys His Leu Trp Phe Phe Leu Leu Leu Val Ala Ala Pro Arg Trp
1               5                   10                  15

Val Leu Ser Gln Val Gln Leu Gln Glu Ser Gly Pro Gly Leu Val Lys
            20                  25                  30

Pro Ser Gly Thr Leu Ser Leu Thr Cys Ala Val Ser Gly Gly Ser Ile
        35                  40                  45

Ser Ser Ser Ile Trp Trp Ser Trp Val Arg Gln Pro Pro Gly Lys Gly
    50                  55                  60

Leu Glu Trp Ile Gly Glu Ile His His Ser Gly Asn Thr Asn Cys Asn
65                  70                  75                  80

Pro Ser Leu Lys Ser Arg Val Thr Ile Ser Ile Asp Lys Ser Lys Asn
                85                  90                  95

Gln Phe Ser Leu Lys Leu Thr Ser Val Thr Ala Ala Asp Thr Ala Val
            100                 105                 110

Tyr Tyr Cys Ala Arg Ala Gly Thr Gly Thr Thr Leu Asp Tyr Trp Gly

        115                 120                 125

Gln Gly Thr Leu Val Thr Val Ser Ser Ala Ser Thr Lys Gly Pro Ser
    130                 135                 140

Val Phe Pro Leu Ala Pro Cys Ser Arg Ser Thr Ser Glu Ser Thr Ala
145                 150                 155                 160

Ala Leu Gly Cys Leu Val Lys Asp Tyr Phe Pro Glu Pro Val Thr Val
                165                 170                 175

Ser Trp Asn Ser Gly Ala Leu Thr Ser Gly Val His Thr Phe Pro Ala
            180                 185                 190

Val Leu Gln Ser Ser Gly Leu Tyr Ser Leu Ser Ser Val Val Thr Val
        195                 200                 205

Pro Ser Ser Asn Phe Gly Thr Gln Thr Tyr Thr Cys Asn Val Asp His
    210                 215                 220

Lys Pro Ser Asn Thr Lys Val Asp Lys Thr Val Glu Arg Lys Cys Cys
225                 230                 235                 240

Val Glu Cys Pro Pro Cys Pro Ala Pro Pro Val Ala Gly Pro Ser Val
                245                 250                 255

Phe Leu Phe Pro Pro Lys Pro Lys Asp Thr Leu Met Ile Ser Arg Thr
            260                 265                 270

Pro Glu Val Thr Cys Val Val Val Asp Val Ser His Glu Asp Pro Glu
        275                 280                 285

Val Gln Phe Asn Trp Tyr Val Asp Gly Val Glu Val His Asn Ala Lys
    290                 295                 300

Thr Lys Pro Arg Glu Glu Gln Phe Asn Ser Thr Phe Arg Val Val Ser
305                 310                 315                 320

Val Leu Thr Val Val His Gln Asp Trp Leu Asn Gly Lys Glu Tyr Lys
                325                 330                 335

Cys Lys Val Ser Asn Lys Gly Leu Pro Ala Pro Ile Glu Lys Thr Ile
            340                 345                 350

Ser Lys Thr Lys Gly Gln Pro Arg Glu Pro Gln Val Tyr Thr Leu Pro
        355                 360                 365

Pro Ser Arg Glu Glu Met Thr Lys Asn Gln Val Ser Leu Thr Cys Leu
    370                 375                 380

Val Lys Gly Phe Tyr Pro Ser Asp Ile Ala Val Glu Trp Glu Ser Asn
385                 390                 395                 400

Gly Gln Pro Glu Asn Asn Tyr Lys Thr Thr Pro Pro Met Leu Asp Ser
                405                 410                 415

Asp Gly Ser Phe Phe Leu Tyr Ser Lys Leu Thr Val Asp Lys Ser Arg
            420                 425                 430

Trp Gln Gln Gly Asn Val Phe Ser Cys Ser Val Met His Glu Ala Leu
        435                 440                 445

His Asn His Tyr Thr Gln Lys Ser Leu Ser Leu Ser Pro Gly Lys
    450                 455                 460

<210>  20
<211>  99
<212>  PRT
<213>  Artificial sequence

<400>  20

Pro Val Ala Gly Pro Ser Val Phe Leu Phe Pro Pro Lys Pro Lys Asp
1               5                   10                  15

Thr Leu Met Ile Ser Arg Thr Pro Glu Val Thr Cys Val Val Val Asp
            20                  25                  30

Val Ser His Glu Asp Pro Glu Val Gln Phe Asn Trp Tyr Val Asp Gly
        35                  40                  45

Val Glu Val His Asn Ala Lys Thr Lys Pro Arg Glu Glu Gln Phe Asn
    50                  55                  60

Ser Thr Phe Arg Val Val Ser Val Leu Thr Val Val His Gln Asp Trp
65                  70                  75                  80

Leu Asn Gly Lys Glu Tyr Lys Cys Lys Val Ser Asn Lys Gly Leu Pro
                85                  90                  95

Ala Pro Ile

<210>  21
<211>  39
<212>  DNA
<213>  Artificial sequence

<220>
<221>  misc_feature
<222>  (19)..(20)
<223>  n is a, c, g, or t

<400>  21
ccaccgtgcc cagcaccann sgtggcagga ccgtcagtc                              39


<210>  22
<211>  44
<212>  DNA
<213>  Artificial sequence

<400>  22
tgcaaggtct ccaacaaagg ctggccagcc cccatcgaga aaac                        44


<210>  23
<211>  54
<212>  DNA
<213>  Artificial sequence


<220>
<221>  misc_feature
<222>  (20)..(20)
<223>  n is a, c, g, or t

<400>  23
acgtgcgtgg tggtggackn gwbgvasrws sacchggagg tccagttcaa ctgg             54

## Description

### Technical field

The present invention belongs to the field of biopharmaceuticals, and specifically relates to a series of variants of Fc regions of human IgG2 antibodies. These variants have enhanced binding affinity to FcyRIIB. Agonistic antibodies comprising these Fc region variants are expected to have better agonistic activity.

### BACKGROUND OF DISCLOSURE

The development of biomacromolecule drugs provides new methods and possibilities for the treatment of a variety of diseases, in particular, molecular targeted therapy based on antibody and heavy chain constant regions (including Fc regions), including antibodies and heavy chain constant region fusion proteins, which have gained great success in the field of biopharmaceuticals and continue to become the focus of this field.

According to different functions, the biomacromolecules can be mainly classified into three categories: effector molecules that clear targets (molecules and cells), blocking molecules that block target-involved signaling pathways, and agonistic molecules that activate signaling pathways downstream of the target. Tumor immunotherapy has gained important breakthroughs in recent years. This benefits from the use of antibodies that increase the activity of immune cells to kill tumors by blocking inhibitory immune checkpoint molecules. However, there are still a large number of cancer patients not responding to existing treatment means. Therefore, on the one hand, it is necessary to optimize the existing tumor immunotherapy means; on the other hand, new tumor immunotherapy drugs need to be developed. It should be noted that there is a class of tumor immunotherapy means referred to as "Agonistic antibodies", which are capable of enhancing the anti-tumor immune response to indirectly kill tumor cells by binding target molecules on the immune cell surface that pass the immune activation signal and activate important immune activation signal pathways controlled by the target molecules. However, while agonistic tumor immunotherapy antibodies have demonstrated their enormous potential in animal models, and have been widely accepted and welcomed as a good tumor immunotherapy concept. However, the research and development of such antibodies have not yet been successful and are a major challenge in the field of tumor immunotherapy. In addition, activation by agonistic antibodies is also an advantageous means of intervention and regulation of key signal pathways of other biological processes, and has a broad application prospect in the field of disease prevention and treatment. For example, activating an immunosuppressive signal pathway may be advantageous for mitigating inflammation and autoimmune symptoms.

An antibody mainly interacts with FcyR through its constant region, and the binding affinity of different antibodies to FcyR also varies, which also affects the function of the antibody *in vivo.* Antibody Fc modification is an important means and trend to optimize the therapeutic antibody, wherein altering the interaction affinity of the Fc region of the antibody to the key protein such as FcyR is a very effective way to optimize the activity of the antibody.

### SUMMARY OF DISCLOSURE

For the above problems, the present invention provides amino acid mutations and combinations capable of enhancing the affinity of Fc to FcyRIIB by performing mutation modification on the Fc region based on IgG; and the Fc containing these amino acid mutations can be used to optimize the activity of the antibody, in particular, to improve the activation activity of the agonistic antibody.

In a first aspect of the present invention, provided herein is a mutant Fc polypeptide fragment having the following features:
(i) said mutant Fc polypeptide fragment has a mutation with respect to a corresponding wild-type Fc fragment before mutation; and
(ii) the affinity of the mutant Fc region to FcyRIIB is increased compared to the wild-type Fc region; and the wild-type Fc is the Fc of wild-type IgG2.

In another preferred embodiment, the mutant Fc polypeptide fragment has a mutation selected from the group consisting of: L328, H268, S267, P271, G327, or a combination thereof, with respect to a corresponding wild-type Fc fragment before mutation;
wherein, the numbering of all amino acids is based on the IgG Eu numbering.

In another preferred embodiment, the affinity may be embodied as a screening enrichment degree or a binding analysis signal.

In another preferred embodiment, the wild-type Fc fragment is residue at 233th to residue at 332th of the amino acid sequence of the IgG2 according to the IgG Eu numbering, and the amino acid sequence of the wild-type Fc fragment is as shown in SEQ ID NO: 20.

In another preferred embodiment, the mutant Fc polypeptide fragment has an enhanced affinity to FcyRIIB as compared to a wild-type Fc fragment.

In another preferred embodiment, the affinity of the mutant Fc polypeptide fragment to FcyRIIB is increased by more than 1 times higher than the affinity of the wild-type Fc fragment to FcyRIIB; preferably, 2, 3, 4, 5, 10, 20, 50, or 100 times higher.

In another preferred embodiment, the affinity of the mutant Fc polypeptide fragment to the activating FcyRs is lower than that to FcyRIIB.

In another preferred embodiment, the affinity of the mutant Fc polypeptide fragment to the FcyRIIB is increased and the affinity of the mutant Fc polypeptide fragment to the activating FcyRs is reduced (I/A ratio is higher than that of the wild-type Fc fragment), as compared to the wild-type Fc fragment.

In another preferred embodiment, the ratio (RIIB/RIIAR) of the affinity of the mutant Fc polypeptide fragment to the FcyRIIB and FcγRIIA^{131R} is higher than that of wild type IgG2.

In another preferred embodiment, the activating FcyRs comprise: FcyRI, FcγRIIA^{131H}, FcγRIIA^{131R}, FcγRIIIA^{158F} and FcγRIIIA^{158V}.

In another preferred embodiment, the mutation L328 includes L328W, L328E, L328Y, L328F, L328M, L328A, L328G, L328N, L328P, L328R, L328V; preferably L328W or L328E.

In another preferred embodiment, the mutation H268 includes H268D, H268S, H268E, H268K, H268N, preferably is H268D, H268S, or H268E.

In another preferred embodiment, the mutation S267 includes S267E, S267V, S267D, S267M, S267Q, S267A, S267G, S267R, S267N, S267W; preferably S267E, S267V, S267D, S267M; more preferably is S267E.

In another preferred embodiment, the mutation P271 includes P271C, P271G, P271V, P271A, P271W, P271Y, P271Q, P271T, P271I, P271L, P271S; preferably is P271C, P271G, P271V, P271A, P271W, P271Y, P271Q, P271T; more preferably is P271C, P271G.

In another preferred embodiment, the mutation G327 includes G327A, G327L, G327S; preferably G327A.

In another preferred embodiment, the mutant Fc polypeptide fragment has a mutation selected from the group consisting of L328W, L328E, H268D, H268S, H268E, S267E, A330S, P233G, P271G, P271C, P271V, P271A, P271W, P271Y, G327A, or a combination thereof, with respect to the wild-type Fc fragment; wherein the numbering of all amino acids is based on the IgG Eu numbering.

In another preferred embodiment, the mutant Fc polypeptide fragment has a mutation in at least one of L328 and H268 with respect to the wild-type Fc fragment, and the specific mutations in the mutant Fc polypeptide fragment are selected from one of the group consisting of: L328W, L328E, H268D, H268S, H268E, H268D/S298L/L328W, S267V/S298L/L328W, V234M/S267E/S298L/L328W, A235W/V266L/S298L/L328W, V234Q/A235G/P238L/S239V/H268D/G327A/L328E/A330S/I332T, S239V/V266L/S298L/L328W, V266L/L328W, V266L/S267D/H268D/E269D/P271Q, S267E/H268S/E269D, P233F/V266L/S298L/L328W, V266L/S298L/L328W, V234Q/A235G/P238L/S239V/G327A/L328E/A330S/I332T, V266L/S267E/H268S/E269V, V266L/S267E/H268S/E269V/P271C, V266L/S267E/H268S/E269A/P271C, S267V/S298G/L328W, V266L/S267M/H268E/P271Q, V266L/S267E/H268S/E269G/P271T, V234Q/V266L/S267D/H268D/E269D/P271Q, V234Q/A235G/P238L/S239V/S267E/H268S/E269D, V266L/S267E/H268S/P271V, V234Q/A235G/P238L/S239V/S267E/S298L/G327A/L328E/A330S/I332T, V234Q/V266L/S267E/H268S/E269V, L328E/I332T; wherein the numbering of all amino acids is based on the IgG Eu numbering.

In another preferred embodiment, the mutant Fc polypeptide fragment has a mutation at S267 with respect to the wild-type Fc fragment, and the specific mutation in the mutant Fc polypeptide fragment is selected from one of the group consisting of: S267E/A330S, S267E/S298G, P233G/S267E, V234M/S267E/S298G/I332L, P233G/S267E/S298G, V266L/S267E/E269K/P271G, P238Q/S267E, S267A/P271C, S267A/P271G, S267E/D270H, S267E/P271C, S267E/P271V, S267E/P271W, S267E/P271Y, S267E/S298R, S267M/P271C, S267M/P271G, S267V/S298L, S267E/P329R; wherein the numbering of all amino acids is based on the IgG Eu numbering.

In another preferred embodiment, the mutant Fc polypeptide fragment has a mutation at S267 with respect to the wild-type Fc fragment, and the specific mutation in the mutant Fc polypeptide fragment is selected from one of the group consisting of: S267E/A330S, S267E/S298G, P233G/S267E, V234M/S267E/S298G/I332L, P233G/S267E/S298G, V266L/S267E/E269K/P271G, S267E/P271C; wherein the numbering of all amino acids is based on the IgG Eu numbering.

In another preferred embodiment, the mutant Fc polypeptide fragment has a mutation at P271 with respect to the wild-type Fc fragment, and the specific mutation in the mutant Fc polypeptide fragment is selected from one of the group consisting of: V266L/S267E/E269K/P271G, V266A/P271C, V266A/P271G, S267A/P271C, S267A/P271G, S267E/P271C, S267E/P271V, S267E/P271W, S267E/P271Y, S267M/P271C, S267M/P271G, V266G/P271C, P271A/S298R, P271G/G236V, P271G/P329S, P271G/P331C, P271G/P331T, P271G/S298D, P271G/S298E, P271G/S298G, P271G/S298K, P271G/S298L, P271G/S298N, P271G/S298R, P271G/T299A, P271G/T299M, P271G/T299S, P271G/T299W, P271G/I332L; wherein the numbering of all amino acids is based on the IgG Eu numbering.

In another preferred embodiment, the mutant Fc polypeptide fragment has a mutation at G327 with respect to the wild-type Fc fragment, and the specific mutation in the mutant Fc polypeptide fragment is selected from one of the group consisting of: G327A/A330R, G327A/A330V, G327A/I332A, G327A/I332C, G327A/I332E.

In another preferred embodiment, the specific mutation of the mutant Fc polypeptide fragment with respect to the wild-type Fc fragment is selected from one of the group consisting of: H268D/S298L/L328W, S267V/S298L/L328W, V234M/S267E/S298L/L328W, A235W/V266L/S298L/L328W, V234Q/A235G/P238L/S239V/H268D/G327A/L328E/A330S/I332T; wherein the numbering of all amino acids is based on the IgG Eu numbering.

In a second aspect of the present invention, provided herein is a mutant immunoglobulin Fc region comprising the mutant Fc polypeptide fragment according to the first aspect of the present invention.

In another preferred embodiment, the immunoglobulin is human IgG2.

In another preferred embodiment, the mutant immunoglobulin Fc region has an enhanced affinity to FcyRIIB as compared to a wild-type IgG2 Fc region.

In another preferred embodiment, the affinity of the mutant immunoglobulin Fc region to FcyRIIB is increased by more than 1 times higher than the affinity of the wild-type IgG2 Fc region to FcyRIIB; preferably, 2, 3, 4, 5, 10, 20, 50, or 100 times higher.

In another preferred embodiment, the affinity may be embodied as a screening enrichment degree or a binding analysis signal.

In another preferred embodiment, the affinity of the mutant immunoglobulin Fc region to an activating FcyR is lower than that to FcyRIIB.

In another preferred embodiment, the affinity of the mutant immunoglobulin Fc region to the FcyRIIB is increased and the affinity of the mutant immunoglobulin Fc region to the activating FcyR is reduced (I/A ratio is higher than that of the wild-type IgG2 Fc region), as compared to the wild-type IgG2 Fc region.

In another preferred embodiment, the ratio (RIIB/RIIAR) of the affinity of the mutant immunoglobulin Fc region to the FcyRIIB and FcγRIIA^{131R} is higher than that of wild type IgG2.

In a third aspect of the present invention, provided herein is an antibody comprising the mutant Fc polypeptide fragment according to the first aspect of the present invention or the mutant immunoglobulin Fc region according to the second aspect of the present invention.

In another preferred embodiment, the antibody is an antibody based on a human IgG2 backbone.

In another preferred embodiment, the antibody is an agonistic antibody.

In another preferred embodiment, the antibody specifically targets a tumor necrosis factor receptor superfamily.

In another preferred embodiment, the antibody is capable of specifically binding to a target selected from the group consisting of CD40, DR5, OX40, CD137, CD27, CD30, GITR, HVEM, TACI, DR4, FAS, or a combination thereof.

In another preferred embodiment, the antibody is capable of specifically binding to OX40.

In another preferred embodiment, the antigen targeted by the antibody is an immune receptor molecule, or a combination thereof.

In another preferred embodiment, the antigen targeted by the antibody is an immunosuppressive receptor molecule, and the immunosuppressive receptor molecule is selected from the group consisting of: PD-1, CTLA-4, VISTA, TIM-3, BTLA, LAG-3, or a combination thereof.

In another preferred embodiment, the antibody is a human antibody, a humanized antibody, or a chimeric antibody.

In another preferred embodiment, the antibody is a monoclonal antibody or a polyclonal antibody, preferably a monoclonal antibody.

In a fourth aspect of the present invention, provided herein is a fusion protein comprising the mutant Fc polypeptide fragment according to the first aspect of the present invention, the mutant immunoglobulin Fc region according to the second aspect of the present invention, or the antibody according to the third aspect of the present invention.

In another preferred embodiment, the fusion protein further comprises other protein sequences having receptor agonist functions or fragments thereof.

In another preferred embodiment, the other protein having a receptor agonist function is a cytokine of the TNF gene family.

In another preferred embodiment, the other protein having a receptor agonist function comprises a ligand molecule of an immune receptor.

In another preferred embodiment, the other protein having the receptor agonist function comprises a ligand molecule of an immune receptor selected from any of the group consisting of: CD80, CD86, ICOSL, OX40L, CD137L, CD40L, CD30L, CD27L, CD244, CD150, CD48, CD84, CD319, Ly118 or CD229, or a combination thereof.

In another preferred embodiment, the other protein having the receptor agonist function comprises a ligand molecule of an immune receptor, and the ligand molecule of the immune receptor is selected from the group consisting of: PD-L1, PD-L2, B7-H3, B7-H4, CD47, VISTA, HVEM, GAL9, or a combination thereof.

In another preferred embodiment, the fusion protein may further comprise a tag sequence useful in expression and/or purification; preferably, the tag sequence comprises a 6His tag, an HA tag, and/or a FLAG tag.

In a fifth aspect of the present invention, provided herein is an isolated polynucleotide encoding the mutant Fc polypeptide fragment according to the first aspect of the present invention, the mutant immunoglobulin Fc region according to the second aspect of the present invention, the antibody according to the third aspect of the present invention, or the recombinant protein according to the fourth aspect of the present invention.

The sixth aspect of the present disclosure provides a vector containing the isolated polynucleotide according to the fifth aspect of the present disclosure.

In another preferred embodiment, the vector is selected from the group consisting of DNAs, RNAs, viral vectors, plasmids, transposons, other gene transfer systems, or combinations thereof.

In another preferred embodiment, the vector comprises a viral vector, such as a vector of a lentivirus, an adenovirus, an AAV virus, a retrovirus, or a combination thereof.

The seventh aspect of the present disclosure provides a host cell containing the vector according to the sixth aspect of the present disclosure, or having the polynucleotide according to the fifth aspect of the present disclosure integrated into the genome of the cell;
or, the host cell expresses the mutant Fc polypeptide fragment according to the first aspect of the present invention, the mutant immunoglobulin Fc region according to the second aspect of the present invention, the antibody according to the third aspect of the present invention, or the recombinant protein according to the fourth aspect of the present invention.

In another preferred embodiment, the host cell comprises a prokaryotic cell or a eukaryotic cell.

In another preferred embodiment, the host cell is selected from the group consisting of: E coli, yeast cells, insect cells, avian cells and mammalian cells.

In an eighth aspect of the present invention, provided herein is a pharmaceutical composition comprising:
(a) the mutant Fc polypeptide fragment according to the first aspect of the present invention, the mutant immunoglobulin Fc region according to the second aspect of the present invention, the antibody according to the third aspect of the present invention, or the recombinant protein according to the fourth aspect of the present invention; and
(b) a pharmaceutically acceptable carrier.

In another preferred embodiment, the pharmaceutical composition may further comprise other drugs for treating tumors, such as cytotoxic drugs.

In another preferred embodiment, the pharmaceutical composition may further comprise other active substances having a receptor agonist function.

In another preferred embodiment, the pharmaceutical composition is an injection dosage form.

In a ninth aspect of the present invention, provided herein is a method of producing the mutant Fc polypeptide fragment according to the first aspect of the present invention, the mutant immunoglobulin Fc region according to the second aspect of the present invention, the antibody according to the third aspect of the present invention, or the recombinant protein according to the fourth aspect of the present invention, comprising the steps of:
(i) culturing the host cell according to the seventh aspect of the invention under suitable conditions to obtain a culture comprising said mutant Fc polypeptide fragment, said mutant immunoglobulin Fc region, said antibody, or said recombinant protein; and
(ii) purifying and/or separating said mutant Fc polypeptide fragment, said mutant immunoglobulin Fc region, said antibody, or said recombinant protein from the culture obtained in step (i).

In another preferred embodiment, the purification can be performed by protein A affinity column purification to obtain a target antibody.

In another preferred embodiment, the purification can be performed by protein G affinity column purification to obtain a target antibody.

In another preferred embodiment, the purity of the target antibody after purification and/or separation is greater than 95%, greater than 96%, greater than 97%, greater than 98%, greater than 99%, preferably 100%.

In a tenth aspect of the present invention, provided herein is use of the mutant Fc polypeptide fragment according to the first aspect of the present invention, the mutant immunoglobulin Fc region according to the second aspect of the present invention, the antibody according to the third aspect of the present invention, the recombinant protein according to the fourth aspect of the present invention, the polynucleotide according to the fifth aspect of the present invention, the vector according to the sixth aspect of the present invention and/or the host cell according to the seventh aspect of the present invention, in the manufacture of a pharmaceutical composition for tumor immunotherapy, reducing inflammation and/or reducing autoimmune symptoms.

In an eleventh aspect of the present invention, provided herein is a method of treating a disease, comprising the steps of: administering to a subject in need thereof the mutant Fc polypeptide fragment according to the first aspect of the present invention, the mutant immunoglobulin Fc region according to the second aspect of the present invention, the antibody according to the third aspect of the present invention, the recombinant protein according to the fourth aspect of the present invention, the polynucleotide according to the fifth aspect of the present invention, the vector according to the sixth aspect of the present invention, the host cell according to the seventh aspect of the present invention, and/or the pharmaceutical composition according to the eighth aspect of the present invention.

In another preferred embodiment, the subject comprises a mammal, preferably is a human.

In another preferred embodiment, the disease is selected from the group consisting of: tumors, inflammatory diseases, autoimmune diseases, or combinations thereof.

In another preferred embodiment, the cancer includes, but is not limited to, breast cancer, prostate cancer, lung cancer, ovarian cancer, cervical cancer, skin cancer, melanoma, colon cancer, gastric cancer, liver cancer, esophageal cancer, renal cancer, throat cancer, thyroid cancer, pancreatic cancer, testicular cancer, brain cancer, bone cancer and blood cancer (such as leukemia, chronic lymphocytic leukemia), etc.

In another preferred embodiment, the cancer includes, but is not limited to, basal cell carcinoma, biliary cancer, bladder cancer, bone cancer, brain and central nervous system (CNS) cancer, cervical cancer, chorionic carcinoma, colorectal cancer, connective tissue cancer, digestive system cancer, endometrial cancer, esophageal cancer, ocular cancer, head and neck cancer, gastric cancer, epithelial neoplasm, renal cancer, throat cancer, liver cancer, lung cancer (small cell, large cell), lymphoma (including Hodgkin lymphoma and non-Hodgkin lymphoma); melanoma; neuroblastoma; oral cancer (e.g., lip, tongue, mouth and pharyngeal); ovarian cancer; pancreatic cancer; retinoblastoma; rhabdomyosarcoma; rectal cancer; respiratory cancer; sarcoma; skin cancer; gastric cancer; testicular cancer; thyroid cancer; uterine cancer; urinary system cancer; and other cancers and sarcoma.

In another preferred embodiment, the method can be used to stimulate an immune response to treat tumors by inhibiting or delaying the growth of a tumor or reducing the size of a tumor.

In another aspect of the present invention, further provided is a method for screening amino acid mutations enhancing protein-molecular binding affinity, the method comprises the following steps:
1) providing a parent protein sequence, introducing amino acid mutations in the parent protein sequence by PCR;
2) constructing the parent protein and the mutated protein into an expression vector to form a mutation library;
3) transfecting the expression vector into mammalian cells, and expressing the parent protein and the mutated protein on the cell surface;
4) incubating labeled interacting molecules and the mammalian cells, and selecting labeled mammalian cells by flow cytometry or magnetic-activated sorting;
5) extracting DNAs from the labeled mammalian cells, sequencing the DNAs, and analyzing the change of the sequence ratios before and after sorting;
wherein, the amino acid mutation in which the ratio after sorting is significantly higher than the ratio before sorting is an amino acid mutation capable of enhancing the binding affinity between the protein and the interacting molecule.

The screening method can efficiently screen various amino acid mutations, has very good convenience and effectiveness, and can be used for screening amino acid mutations which enhance various protein-protein interactions or protein-other molecules interactions.

The beneficial effects of the present invention include: the modified Fc region or Fc fragment of the present invention has an enhanced affinity to FcyRIIB, and some of them have a reduced affinity to activating FcyRs while having the enhanced affinity to FcyRIIB. These proteins can be used to design, modify, or optimize the activation activity of antibodies or fusion proteins. In particular, it is possible to design agonist activity of an antibody or a fusion protein based on a human IgG2 backbone. Thus, it is possible to provide proteins with significantly enhanced agonistic activity, for the treatment of tumors, inflammatory diseases, autoimmune diseases, or a combination thereof, which have a significant application prospect.

### BRIEF DESCRIPTION OF DRAWINGS

FIG. 1 shows the construction of a plasmid for displaying antibodies on the surface of mammalian cells.
FIG. 2 shows a schematic diagram of a mutation screening process. Specifically, the process comprises: mutation library is constructed and transferred to the cells by retrovirus; the Fc mutant library is expressed on the cell surface; the cells with high affinity to FcyRIIB are sorted; the DNA is extracted and sequenced by high-throughput sequencing (NSG); the enrichment of various mutations after sorting relative to before sorting are analyzed, and the mutations with relatively high enrichment factor compared with the wild type are selected.
FIG. 3 shows the Eu numbering of CH2 amino acids on human IgG2 Fc.
FIG. 4 shows that flow cytometry sorting can gradually enrich cells with high binding affinity to FcyRIIB. The cell flow cytometry results of wild-type human IgG2 cells and human IgG2 library LibraryMix cells after one or two rounds of FcyRIIB sorting are shown, respectively.
FIG. 5 shows that the binding affinity of the cells screened from the library to FcyRIIB is significantly improved. The cell flow cytometry results of wild-type human IgG2 cells and cell libraries after two rounds of FcyRIIB sorting are shown, respectively.
FIG. 6 shows a schematic diagram of two types of combined mutation libraries. Type I: combined point mutation among four regions, i.e., regions P233-V240, V266-P271, S298-T299 and G327-I332 each has 0-1 mutation, and the combined mutation contains 0-4 point mutations. Type II: combined mutation of multiple amino acids in one region, i.e., regions P233-V240, V266-P271, S298-T299 or G327-I332 has combined mutation inside each region (respectively 0-7, 0-6, 0-2 and 0-6 combined mutations).
FIG. 7 shows the flow cytometry results of the library IgG2_C01 and the library IgG2_C02 after flow cytometry sorting and the wild-type IgG2. The ratio of the cells binding to FcγRIIB in the library IgG2_C01 and the library IgG2_C02 is improved.
FIG. 8 shows the binding affinity of library Cmix and library C2 cells to FcyRIIB before ("_befor") and after ("_1^{st}" and "_2^{nd}") flow cytometry sorting. The results show that the ratio of cells binding to FcyRIIB in the sorted library was gradually increased (2^{nd} > 1^{st} > befor).
FIG. 9 shows the binding affinity of different IgG2 antibody Fc mutants to FcyRI (expressed as ELISA signal: absorbance at 650 nm (A650)).
FIG. 10 shows the binding affinity of different IgG2 antibody Fc mutants to FcγRIIA^{131H} (expressed as ELISA signal: absorbance at 650 nm (A650)).
FIG. 11 shows the binding affinity of different IgG2 antibody Fc mutants to FcγRIIA^{131R} (expressed as ELISA signal: absorbance at 650 nm (A650)).
FIG. 12 shows the binding affinity of different IgG2 antibody Fc mutants to FcyRIIB (expressed as ELISA signal: absorbance at 650 nm (A650)).
FIG. 13 shows the binding affinity of different IgG2 antibody Fc mutants to FcγRIIIA^{158F} (expressed as ELISA signal: absorbance at 650 nm (A650)).
FIG. 14 shows the binding affinity of different IgG2 antibody Fc mutants to FcγRIIIA^{158V} (expressed as ELISA signal: absorbance at 650 nm (A650)).
FIG. 15 shows the binding affinity of the IgG2 mutants corresponding to IgG1 V11(hIgG1V11) to FcγRIIA^{131H} and FcyRIIB by ELISA (expressed as ELISA signal: absorbance at 650 nm (A650)).
FIG. 16 shows the immune activation activity analysis results of different antibody Fc mutant anti-human OX40 antibodies, expressed as a column diagram of the mean fluorescence intensity of CD4 positive cell CFSE (the lower the CFSE signal, the higher the activity).
FIG. 17 shows the immune activation activity analysis results of different antibody Fc mutant anti-human OX40 antibodies, expressed as the fluorescence intensity histogram of CD4 positive cell CFSE (the lower the CFSE signal, the higher the activity).

### DETAILED DESCRIPTION

Unless otherwise stated, the scientific and technical terms used herein shall be understood as having the meanings as commonly understood by one of ordinary skills in the art. Unless otherwise required, as used herein, terms in singular forms include the meaning of plurality, and vice versa. In general, as used herein, nomenclatures and techniques in the cell and tissue culturing/incubation, molecular biology, immunology, protein and nucleic acid chemistry described herein are those already known and commonly used in the art.

Methods and techniques used in the present invention are generally performed as in conventional practices and as described in general and specialized references cited and discussed in this disclosure, unless otherwise indicated. For example, reference can be made to the followings: Molecular Cloning: A Laboratory Manual, Sambrook et al., the 2ndEdition, Cold Spring Harbor Laboratory Press, Cold Spring Harbor, N.Y., 1989; Current Protocols in Molecular Biology, Ausubel et al. Greene Publishing Associates, 1992; Antibodies: A Laboratory Manual, Harlow and Lane, Cold Spring Harbor Laboratory Press, Cold Spring Harbor, N.Y., 1990, all being incorporated by reference in their entirety. Enzymatic reactions and purifications are performed by following manufacturers' instructions or as known in the art or as specified in the present disclosure. As used herein, nomenclatures, test methods and techniques in biology, pharmacology, medicine and pharmaceutical chemistry are those already known and commonly used in the art. Chemical synthesis, chemical analysis, pharmaceutical manufacturing, formulation and delivery and patient treatment all follow standard practices.

Unless otherwise stated, the terms have the meanings as defined below.

An "antibody" (Ab) should include, but is not limited to, a glycoprotein immunoglobulin or antigen-binding portions thereof. Antibodies specifically bind to corresponding antigens, and each comprises at least two heavy (H) chains and two light (L) chains that bind to one another through disulfide bonds. Each heavy chain comprises a heavy chain variable region (VH) and a heavy chain constant region. The "heavy chain constant region" or CDs of antibodies comprise three domains identified as CH1, CH2 and CH3, as well as a hinge region between the CH1 domain and the CH2 domain. Each light chain comprises a light chain variable region (VL) and a light chain constant region. The light chain constant region consists of one domain CL. VH and VL can each be subdivided into hypervariable regions called "complementary determining regions (CDRs)" and the interspersed relatively conserved regions called "frame regions (FRs)". VH and VL are each composed of three CDRs and four FRs as arranged in the following order from the amino end to the carboxyl end: FR1, CDR1, FR2, CDR2, FR3, CDR3, FR4. The heavy chain and the light chain variable regions provide the domains that interact with antigens.

"Fc region" (crystallizable fragment region) or "Fc domain" or "Fc" refers to the C-terminal portion of the heavy chain, which mediates the binding between the immunoglobulin and host tissues or factors, including the binding to Fc receptors on various immune cells (e.g., effector cells) or to the first component of the classic complement system(Clq). Thus, the Fc region is a polypeptide that forms the part of the antibody heavy chain constant region other than the first constant region immunoglobulin domain (CH1 domain). For the allotypes of IgG, IgA and IgD antibodies, the Fc region is composed of two identical protein fragments from CH2 and CH3 domains of the two heavy chains; and in IgM and IgE, the Fc region comprises three heavy chain constant domains (CH2-CH3-CH4 domains) in each polypeptide chain. For IgG, the Fc region contains the hinge region between the immunoglobulin domains CH2 and CH3 and the hinge region between CH1 and CH2. Though boundaries of Fc regions of heavy chains of immunoglobulins are variable, Fc regions in heavy chains of human IgGs are normally defined as the segment spanning position P231 to the carboxyl end of the heavy chains, as numbered according to the EU index, the same as in the Kabat system. The CH2 domain of the human IgG Fc region extends from about amino acid 231 to about amino acid 340, while the CH3 domain is located at the C-terminal of the CH2 domain of the Fc region, i.e., it extends from about amino acid 341 of IgG to about amino acid 447.

The "Fc polypeptide fragment" refers to a fragment contained in the Fc region. The Fc polypeptide fragment herein refers to a fragment extending from the amino acid 233 to the amino acid 332 in the Fc region, or other fragments within the Fc region containing said fragment, such as the Fc CH2 domain or the Fc region.

As used herein, "Fc region" or "Fc polypeptide fragment" may be a natural Fc or a modified Fc. In addition, "Fc region" or "Fc polypeptide fragment" may also refer to such area in a separated state, or such area provided in a protein polypeptide comprising Fc, which is also referred to as an "Fc fusion protein" (e.g., an antibody or an immunoadhesin).

"Fc receptor" or "FcR" is the receptor binding to the Fc region of an immunoglobulin. FcRs binding to IgG antibodies include the FcyR family, including their allelic variants and alternative splicing variants. Members of the human Fcγ receptor family include FcyRI (CD64), FcyRIIA (CD32a), FcyRIIB (CD32b), FcyRIIIA (CD 16a), FcyRIIIB (CD16b). Therein, FcyRIIB is the only inhibitory Fcγ receptor, and the rest are all activating Fcγ receptors. Most natural effector cells co-express one or more activating FcyRs and the inhibitory FcyRIIB, while natural killer (NK) cells selectively express an activating Fcγ receptor (FcyRIII in mice, FcyRIIIA in humans), while not the inhibitory FcyRIIB in mice and humans. These Fcγ receptors have different molecular structures and therefore different affinity to subclasses of IgG antibodies. Among these Fcγ receptors, FcyRI is a high-affinity receptor, while FcyRIIA, FcyRIIB and FcyRIIIA are low-affinity receptors. Genetic polymorphism is seen in these different Fcγ receptors, which also influences binding affinity. Most dominant genetic polymorphisms include the R131/H131 in FcyRIIA and the V158/F158 in FcyRIIIA. Some of these polymorphisms are found disease-associated. And, responsiveness to certain therapeutic antibodies depends on the presence of certain polymorphisms in Fcγ receptors.

As used herein, a "sequence" should be understood as encompassing sequences that are substantially identical to a specified one according to the invention. The term "substantially identical sequences" refers sequences sharing a sequence identity of at least 70, 75 or 80%, preferably at least 90 or 95%, more preferably at least 97, 98 or 99% in the optimal alignment using, for example, GAP or BESTFIT and the default gap value. Preferably, the differential residues are conservative amino acid substitution. The term "conservative amino acid substitution" refers to substitution between amino acid residues having similar chemistry (e.g., charge or hydrophilicity) in the side-chain R group. Generally, conservative amino acid substitution does not substantially change a protein's functional properties. When difference(s) between two or more amino acid sequences reside(s) in conservative substitution(s), the sequence identity percentage or similarity value can be up-adjusted to correct for the conservativeness of substitution. See, for example, Pearson, Methods Mol. Biol. 243: 307-31(1994). Examples of amino acids having side-chains with similar chemistry include: 1) aliphatic side chains, including glycine, alanine, valine, leucine and isoleucine; 2) aliphatic-hydroxy side chains, including serine and threonine; 3) amide-containing side chains, including asparagine and glutamine; 4) aromatic side chains, including phenylalanine, tyrosine and tryptophan; 5) basic side chains, including lysine, arginine and histidine; 6) acidic side chains, including aspartic acid and glutamic acid; and 7) sulfur-containing side chain, including cysteine and methionine. Preferred conservative amino acid substitutions include valine-leucine-isoleucine, phenylalanine-tyrosine, lysine-arginine, alanine-valine, glutamate-aspartate, and asparagine-glutamine. As noted above, in addition to the amino acid mutations provided by the present invention, the Fc region, the antibody, the Fc fragment or the fusion protein according to the present invention may further comprise other possible modifications not disclosed in the present invention as long as the "substantially identical" requirements described above are met.

Amino acids of the antibody and a fragment or domain thereof according to the invention are numbered according to the Eu numbering system for IgGs. The mutant types of some mutants involved in the present invention and their nomenclature are shown in Table 1.

Antibodies usually specifically bind to the corresponding antigens with a high affinity (likewise, the proteins of the invention may also specifically bind to their epitope recognition molecules), as indicated by, for example, a dissociation constant (KD) of 10⁻⁵-10⁻¹¹M or less. A KD above about 10⁻⁴ M⁻¹ is usually understood as indicating a nonspecific binding. In this disclosure, an antibody "specifically binding to" an antigen means that the antibody binds to the antigen or a substantially identical antigen with a high affinity as measured by a KD of 10⁻⁷M or less, preferably 10⁻⁸M or less, more preferably 5×10⁻⁹M or less and most preferably 10⁻⁸-10⁻¹⁰M or less, and does not bind to any irrelevant antigen with a significant affinity. An antigen is "substantially identical" to a specified antigen, if they share a high sequence identity, such as a sequence identity of at least 80%, at least 90%, preferably at least 95%, more preferably at least 97%, or even more preferably at least 99%.

Immunoglobulins may come from any commonly known isoform. IgG isoforms can be subdivided into subclasses in some species: IgG1, IgG2, IgG3 and IgG4 in human, IgG1, IgG2a, IgG2b, and IgG3 in mouse. The term "isotype" refers to antibodies encoded by heavy chain constant region genes (e.g. IgM or IgG1). The "antibodies" include naturally occurring and non-naturally occurring antibodies, monoclonal and polyclonal antibodies, chimeric and humanized antibodies, human and non-human antibodies, wholly synthetic antibodies, and single chain antibodies. The "natural IgG" described in the present invention refers to those IgG that can be naturally present, the sequences of which are not artificially modified. It should be noted that, due to the polymorphism of the gene, even if the natural IgG may also have different mutants. Any non-artificially modified IgG in the natural state is referred to as natural IgG, whether there is a mutation or not.

An "agonistic antibody" is an antibody that binds to and activates a receptor. Examples of such agonism include an agonistic antibody that binds to a receptor of a tumor necrosis factor receptor (TNFR) superfamily and induces cell apoptosis expressing a TNF receptor. Assays for determining apoptosis-inducing effects are described in WO98/51793 and WO99/37684, which are particularly incorporated herein by reference. In a specific embodiment of the present invention, the anti-CD40 agonistic antibody is capable of enhancing the anti-tumor immune response to indirectly kill tumor cells by binding to the target molecule transmitting the immune activation signal on the immune cell surface and activating the important immune activation signal pathway controlled by the target molecule. Examples of agonistic antibodies that have entered the clinical study stage may be referred to in PCT/CN2017/087620.

The term "agonistic activity" refers to the activity of inducing certain specific physiological activity changes by binding an antibody to an antigen molecule and exciting the antigen molecule to generate a signal. Antigen molecules include receptor molecules and other molecules with signal or physiological functions. Said specific physiological activities include but are not limited to: proliferation activity, survival activity, differentiation activity, transcriptional activity, membrane transport activity, affinity, protein decomposition activity, phosphorylation/dephosphorization activity, redox activity, transfer activity, nucleic acid decomposition activity, dehydration activity, cell death induction activity, apoptosis induction activity, and the like.

The term "parent" refers to an object that is modified during protein modification. The parent may be a naturally occurring polypeptide, or a variant or modified version of a naturally occurring polypeptide. In some embodiments, the parental Fc region of the present invention is an Fc region of natural IgG.

A "variant" refers to a protein obtained after modification of a parental protein in a protein modification process. Specifically, variants can be proteins that are derived from the parental protein by mutations, deletions and/or additions of amino acids in the parental protein, and retain some or all of the function of the parent. Preferably, sequence of a mutant herein has at least about 80% homology to the parent's sequence, most preferably at least about 90% homology, more preferably at least about 95% homology. Accordingly, the term "Fc variant" as used herein refers to an Fc sequence that differs from the parent Fc sequence due to at least one amino acid modification. The Fc variant may comprise only the Fc region, or may be present in the context of an antibody, Fc fusion, isolated Fc, Fc region, or other polypeptide that is substantially encoded by Fc. The Fc variant may refer to the Fc polypeptide itself, a composition comprising an Fc variant polypeptide, or an amino acid sequence encoding it.

As used herein, "tumor necrosis factor receptor superfamily" and "TNF receptor superfamily" both refer to receptor polypeptides that bind to cytokines of the TNF family. Generally, these receptors are type I transmembrane receptors comprising one or more cysteine-rich repeats in their extracellular domain. Examples of cytokines of TNF family include tumor necrosis factor-α (TNF-α), tumor necrosis factor-β (TNF-β or lymphotoxin), CD30a ligand, CD27a ligand, CD40a ligand, OX-40a ligand, 4-1BB ligand, Apo-1 ligand (also known as Fas ligand or CD95 ligand), Apo-2 ligand (also known as TRAIL), Apo-3 ligand (also known as TWEAK), osteoprotegerin (OPG), APRIL, RANK ligand (also known as TRANCE) and TALL-1 (also known as BlyS, BAFF or THANK). Examples of receptors of the TNF receptor superfamily include type 1 tumor necrosis factor receptor (TNFR1), type 2 tumor necrosis factor receptor (TNFR2), p75 nerve growth factor receptor (NGFR), B-cell surface antigen CD40, T-cell antigen OX-40, Apo-1 receptor (also known as Fas or CD95), Apo-3 receptor (also known as DR3, sw1-1, TRAMP and LARD), receptors called "transmembrane activator and CAML-interactor" or "TACI", BCMA protein, DR4, DR5 (also known as Apo-2; TRAIL-R2, TR6, Tango-63, hAPO8, TRICK2 or KILLER), DR6, DcR1 (also known as TRID, LIT or TRAIL-R3), DcR2 (also known as TRAIL-R4 or TRUNDD), OPG, DcR3 (also known as TR6 or M68), CAR1, HVEM (also known as ATAR or TR2), GITR, ZTNFR-5, NTR-1, TNFL1, CD30, lymphotoxin β receptor (LTBr), 4-1BB receptor and TR9 (EP988,371 A1).

As used herein, the terms "ratio of the affinity to the inhibitory Fcγ receptor to the affinity to the activating Fcγ receptor" and "I/A ratio" both refer to the ratio of the antibody heavy chain constant region's affinity to the inhibitory Fcγ receptor (such as human FcyRIIB) to its highest affinity to the activating Fcγ receptor of the same species, e.g., human FcyRI, FcyRIIA, FcyRIIIA, FcyRIIIB.

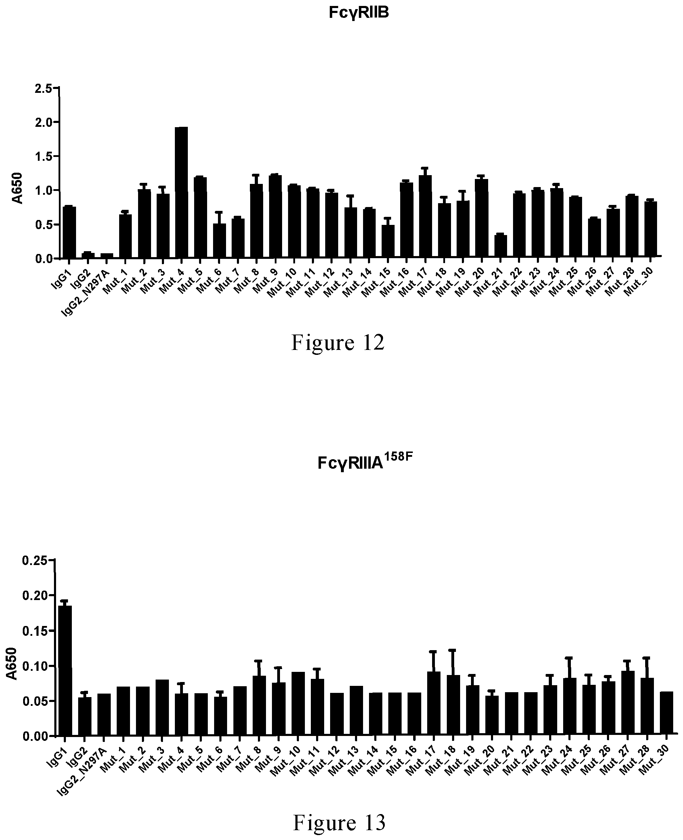

The term "affinity" refers to the capability of two molecules binding to each other, as usually measured by a KD value. Affinity herein can also be measured by an enrichment factor (as shown in Table 7) or binding analysis signal (as shown in FIG. 12). The term "KD" refers to the equilibrium dissociation constant of interaction between two molecules (e.g., a specific antibody vs. an antigen or a specific ligand vs. a receptor). The term "enrichment factor" refers to the ratio of the percentage of the cells expressing the antibody containing the specific Fc mutation after sorting to such percentage before sorting (analyzed by second-generation sequencing, as shown in Table 7), and the sorting is flow cytometry sorting or magnetic-activated sorting provided by the present invention. The "binding analysis signal" is the absorbance value in the ELISA analysis provided by the present invention (as shown in FIG. 12).

"Human" antibodies refer to such antibodies wherein the variable regions comprise FRs and CDRs from human immunoglobulins. And, the antibody may also comprise a constant region from a human immunoglobulin, if present. A human antibody according to the invention may include amino acid residues not encoded by human immunoglobulin sequences, such as mutations introduced by random or site-directed mutagenesis in vitro or somatic mutation in vivo. However, as used herein, the term "human antibody" has no intention to include such antibodies that comprise non-human mammalian (e.g., mouse) CDR sequences grafted between human frame sequences. The term "human" antibody and "wholly human" antibody are used as synonymous to each other.

The "antibodies" of the invention include naturally occurring and non-naturally occurring antibodies, monoclonal and polyclonal antibodies, chimeric and humanized antibodies, human and non-human antibodies and wholly synthetic antibodies.

A "humanized" antibody refers to an antibody wherein some, majority or all of the amino acids outside the non-human CDR domains are replaced by corresponding amino acids from a human immunoglobulin. In an embodiment of a humanized antibody, some, majority or all of the amino acids outside the CDR domains are replaced by corresponding amino acids from a human immunoglobulin, and some, majority or all of the amino acids in one or more of the CDRs remain unchanged. Minor addition, deletion, insertion, substitution or modification of amino acid(s) is tolerable, as long as it does not destroy the antibody's capability of binding to the corresponding antigen. A "humanized" antibody retains the antigen binding specificity of the original antibody.

A "chimeric antibody" refers to an antibody wherein the variable region(s) come(s) from a species and the constant region(s) from another, for example, an antibody in which the variable region(s) may come from a mouse antibody and the constant region(s) from a human antibody.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in Sambrook, Molecular Cloning: A Laboratory Manual (New York: Cold Spring Harbor Laboratory Press, 1989), or followed the manufacturer's recommendation. Unless otherwise stated, the percentages and parts are by weight.

**Table 1. Mutation types of some mutants involved in the present invention and their nomenclature**

| Name | IgG2 mutation | Name | IgG2 mutation |
|---|---|---|---|
| Mut_1 | L328W | Mut_16 | S267E/S298G |
| Mut_2 | H268D/S298L/L328W | Mut_17 | V266L/S267E/H268S/E269V |
| Mut_3 | S267V/S298L/L328W | Mut_18 | P233G/S267E |
| Mut_4 | V234M/S267E/S298L/L328W | Mut_19 | V266L/S267E/H268S/E269V/P271C |
| Mut_5 | A235W/V266L/S298L/L328W | Mut_20 | V266L/S267E/H268S/E269A/P271C |
| Mut_6 | V234Q/A235G/P238L/S239V/H26 8D/G327A/L328E/A330S/I332T | Mut_21 | S267V/S298G/L328W |
| Mut_7 | S239V/V266L/S298L/L328W | Mut_22 | V266L/S267M/H268E/P271Q |
| Mut_8 | V266L/L328W | Mut_23 | V266L/S267E/E269K/P271G |
| Mut_9 | V266L/S267D/H268D/E269D/P27 1Q | Mut_24 | V266L/S267E/H268S/E269G/P271T |
| Mut_10 | S267E/H268S/E269D | Mut_25 | V234Q/V266L/S267D/H268D/E269D/P 271Q |
| Mut_11 | S267E/A330S | Mut_26 | V234Q/A235G/P238L/S239V/S267E/H2 68S/E269D |
| Mut_12 | P233F/V266L/S298L/L328W | Mut_27 | V266L/S267E/H268S/P271V |
| Mut_13 | V266L/S298L/L328W | Mut_28 | P233G/S267E/S298G |
| Mut_14 | V234M/S267E/S298G/I332L | Mut_29 | V234Q/A235G/P238L/S239V/S267E/S2 98L/G327A/L328E/A330S/I332T |
| Mut_15 | V234Q/A235G/P238L/S239V/G32 7A/L328E/A330S/I332T | Mut_30 | V234Q/V266L/S267E/H268S/E269V |

### Materials and methods

### Construction of vector for mammalian cell surface display

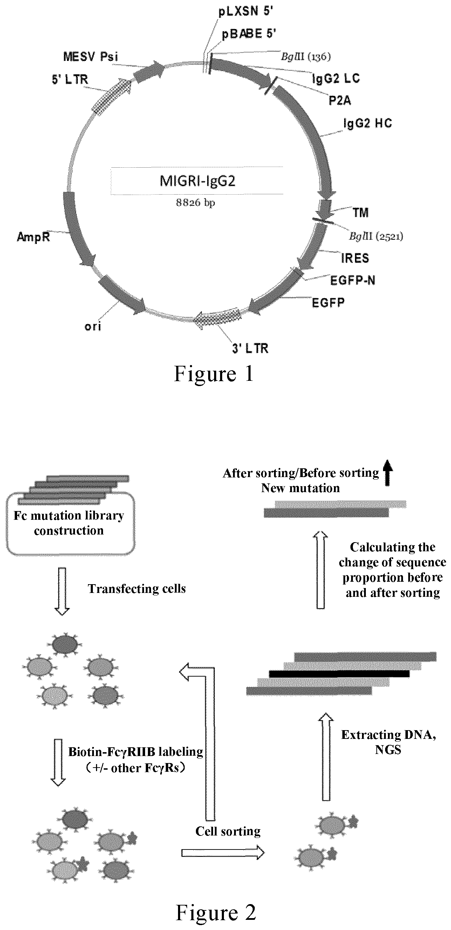

The light and heavy-chain full-length sequences of human IgG2 anti-mouse CD40 antibody are constructed to retrovirus vector MIGRI (Addgene, Pear et al. Blood. 1998 Nov 15. 92(10):3780-92) by Multi One Step Cloning Kit (Yeasen Biotechnology (Shanghai) Co., Ltd., China). The primers used are shown in Table 2, and the construction is shown in Figure 1. The vector is digested by restriction endonuclease BglII, into which light chain DNA and heavy chain DNA (connected by a self-cleaving Linker P2A) of the complete antibody are inserted; The end of the heavy chain DNA comprises a transmembrane domain DNA (DNA sequence of each fragment is shown in Table 3). The plasmid expresses the GFP protein while expressing the antibody human IgG2. The amino acid sequences of the surface display antibody and the related Fc region thereof are as shown in Table 4. The construct is verified by sequencing.

**Table 2. Primers used in construction of antibody-expressing vectors for mammalian cell surface display**

| | | |
|---|---|---|
| SEQ ID NO: 1 | 5MIGR1-BglI I-LC | CTAGGCGCCGGAATTAGATCTACCATGGAGA CAGACAGAC |
| SEQ ID NO: 2 | 3'LC-P2 | GTTTGTTGCGCCGGATCCACACTCTCCCCTGT TGAA |
| SEQ ID NO: 3 | 5'LC-P2 | TTCAACAGGGGAGAGTGTGGATCCGGCGCA ACAAAC |
| SEQ ID NO: 4 | 3'P2-HC | GCTGAGCCTGATGTCCATGGGTCCAGGATTC TCTTC |
| SEQ ID NO: 5 | 5'P2-HC | GAAGAGAATCCTGGACCCATGGACATCAGG CTCAGC |
| SEQ ID NO: 6 | 3'HC-TM | GGATGGAGGAGGCTCGAGTTTACCCGGAGA CAGGGA |
| SEQ ID NO: 7 | 5'HC-TM | TCCCTGTCTCCGGGTAAACTCGAGCCTCCTCC ATCC |
| SEQ ID NO: 8 | 3MIGR1-BglI I-TM | TTCGTTAACCTCGAGAGATCTTCACGCTAGA GAATGAGG |

**Table 3. DNA sequence of each fragment of the surface displayed antibodies**

| |
|---|
| Antibody light chain DNA sequence (SEQ ID NO:9) |
| ATGGAGACAGACAGACTCCTGCTATGGGTGCTGCTGCTCTGGGTGC CAGGCTCCACTGGTGACACTGTACTGACCCAGTCTCCTGCTTTGGCT GTGTCTCCAGGAGAGAGGGTTACCATCTCCTGTAGGGCCAGTGACA GTGTCAGTACACTTATGCACTGGTACCAACAGAAACCAGGACAGCA ACCCAAACTCCTCATCTATCTAGCATCACACCTAGAATCTGGGGTCC CTGCCAGGTTCAGTGGCAGTGGGTCTGGGACAGACTTCACCCTCAC CATTGATCCTGTGGAGGCTGATGACACTGCAACCTATTACTGTCAGC AGAGTTGGAATGATCCGTGGACGTTCGGTGGAGGCACCAAGCTGGA ATTGAAACGAACTGTGGCTGCACCATCTGTCTTCATCTTCCCGCCAT CTGATGAGCAGTTGAAATCTGGAACTGCCTCTGTTGTGTGCCTGCTG AATAACTTCTATCCCAGAGAGGCCAAAGTACAGTGGAAGGTGGATA ACGCCCTCCAATCGGGTAACTCCCAGGAGAGTGTCACAGAGCAGGA CAGCAAGGACAGCACCTACAGCCTCAGCAGCACCCTGACGCTGAGC AAAGCAGACTACGAGAAACACAAAGTCTACGCCTGCGAAGTCACCC ATCAGGGCCTGAGCTCGCCCGTCACAAAGAGCTTCAACAGGGGAGA GTGT |
| Self-cleaving Linker P2A DNA sequence (SEQ ID NO:10) |
| GGATCCGGCGCAACAAACTTCTCTCTGCTGAAACAAGCCGGAGATG TCGAAGAGAATCCTGGACCC |
| Antibody heavy chain DNA sequence (SEQ ID NO:11) |
| ATGGACATCAGGCTCAGCTTGGTTTTCCTTGTCCTTTTCATAAAGG TGTCCAGTGTGAGGTGCAGCTGGTGGAGTCTGATGGAGGCTTAGTG CAGCCTGGAAGGTCCCTAAAACTCCCCTGTGCAGCCTCAGGATTCA CTTTCAGTGACTATTACATGGCCTGGGTCCGCCAGGCTCCAACGAAG GGGCTGGAGTGGGTCGCAAGCATTAGTTATGATGGTAGTAGCACTT ACTATCGAGACTCCGTGAAGGGCCGATTCACTATCTCCAGAGATAA TGCAAAAAGCACCCTATACCTGCAAATGGACAGTCTGAGGTCTGAG GACACGGCCACTTATTACTGCGGAAGACACAGTAGCTACTTTGATT ACTGGGGCCAAGGAGTCATGGTCACAGTCTCGAGCGCCTCCACCAA GGGCCCATCGGTCTTCCCCCTGGCGCCCTGCTCCAGGAGCACCTCCG AGAGCACAGCGGCCCTGGGCTGCCTGGTCAAGGACTACTTCCCCGA ACCGGTGACGGTGTCGTGGAACTCAGGCGCTCTGACCAGCGGCGTG CACACCTTCCCAGCTGTCCTACAGTCCTCAGGACTCTACTCCCTCAG CAGCGTGGTGACCGTGCCCTCCAGCAACTTCGGCACCCAGACCTAC ACCTGCAACGTAGATCACAAGCCCAGCAACACCAAGGTGGACAAG ACAGTTGAGCGCAAATGTTGTGTCGAGTGCCCACCGTGCCCAGCAC CACCTGTGGCAGGACCGTCAGTCTTCCTCTTCCCCCCAAAACCCAAG GACACCCTCATGATCTCCCGGACCCCTGAGGTCACGTGCGTGGTGGT GGACGTGAGCCACGAAGACCCCGAGGTCCAGTTCAACTGGTACGTG GACGGCGTGGAGGTGCATAATGCCAAGACAAAGCCACGGGAGGAG CAGTTCAACAGCACGTTCCGTGTGGTCAGCGTCCTCACCGTTGTGCA CCAGGACTGGCTGAACGGCAAGGAGTACAAGTGCAAGGTCTCCAAC AAAGGCCTCCCAGCCCCCATCGAGAAAACCATCTCCAAAACCAAAG GGCAGCCCCGAGAACCACAGGTGTACACCCTGCCCCCATCCCGGGA GGAGATGACCAAGAACCAGGTCAGCCTGACCTGCCTGGTCAAAGGC TTCTACCCCAGCGACATCGCCGTGGAGTGGGAGAGCAATGGGCAGC CGGAGAACAACTACAAGACCACACCTCCCATGCTGGACTCCGACGG CTCCTTCTTCCTCTACAGCAAGCTCACCGTGGACAAGAGCAGGTGGC AGCAGGGGAACGTCTTCTCATGCTCCGTGATGCATGAGGCTCTGCA CAACCACTACACGCAGAAGAGCCTCTCCCTGTCTCCGGGTAAA |
| Transmembrane domain DNA sequence (SEQ ID NO:12) |
| CTCGAGCCTCCTCCATCCACTGTCTCCAACATGGCGACCGTTGCTGT TCTGGTTGTCCTTGGAGCTGCAATAGTCACTGGAGCTGTGGTGGCTT TTGTGATGAAGATGAGAAGGAGAAACACAGGTGGAAAAGGAGGGG ACTATGCTCTGGCTCCAGGCTCCCAGACCTCTGATCTGTCTCTCCCA GATTGTAAAGTGATGGTTCATGACCCTCATTCTCTAGCGTGA |

**Table 4. Amino acid sequence of the surface display antibody and the related Fc region thereof**

| |
|---|
| Amino acid sequence of the human IgG2 anti-mouse CD40 surface displaye d antibody (SEQ ID NO: 13): |
| METDRLLLWVLLLWVPGSTGDTVLTQSPALAVSPGERVTISCRASDSVSTL MHWYQQKPGQQPKLLIYLASHLESGVPARFSGSGSGTDFTLTIDPVEADDT ATYYCQQSWNDPWTFGGGTKLELKRTVAAPSVFIFPPSDEQLKSGTASVV CLLNNFYPREAKVQWKVDNALQSGNSQESVTEQDSKDSTYSLSSTLTLSK ADYEKHKVYACEVTHQGLSSPVTKSFNRGECGSGATNFSLLKQAGDVEE NPGPMDIRLSLVFLVLFIKGVQCEVQLVESDGGLVQPGRSLKLPCAASGFT FSDYYMAWVRQAPTKGLEWVASISYDGSSTYYRDSVKGRFTISRDNAKST LYLQMDSLRSEDTATYYCGRHSSYFDYWGQGVMVTVSSASTKGPSVFPL APCSRSTSESTAALGCLVKDYFPEPVTVSWNSGALTSGVHTFPAVLQSSGL YSLSSVVTVPSSNFGTQTYTCNVDHKPSNTKVDKTVERKCCVECPPCPAPP VAGPSVFLFPPKPKDTLMISRTPEVTCVVVDVSHEDPEVQFNWYVDGVEV HNAKTKPREEQFNSTFRVVSVLTVVHQDWLNGKEYKCKVSNKGLPAPIEK TISKTKGQPREPQVYTLPPSREEMTKNQVSLTCLVKGFYPSDIAVEWESNG QPENNYKTTPPMLDSDGSFFLYSKLTVDKSRWQQGNVFSCSVMHEALHN HYTQKSLSLSPGKLEPPPSTVSNMATVAVLVVLGAAIVTGAVVAFVMKM RRRNTGGKGGDYALAPGSQTSDLSLPDCKVMVHDPHSLA |
| Amino acid sequence of the Fc region of the human IgG2 anti-mouse CD4 0 surface displayed antibody (SEQ ID NO: 14): |
| APPVAGPSVFLFPPKPKDTLMISRTPEVTCVVVDVSHEDPEVQFNWYVDG VEVHNAKTKPREEQFNSTFRVVSVLTVVHQDWLNGKEYKCKVSNKGLPA PIEKTISKTKGQPREPQVYTLPPSREEMTKNQVSLTCLVKGFYPSDIAVEWE SNGQPENNYKTTPPMLDSDGSFFLYSKLTVDKSRWQQGNVFSCSVMHEA LHNHYTQKSLSLSPGK |
| Amino acid sequence of the Fc CH2 domain of the human IgG2 anti-mouse CD40 surface displayed antibody (SEQ ID NO: 15): |
| APPVAGPSVFLFPPKPKDTLMISRTPEVTCVVVDVSHEDPEVQFNWYVDG VEVHNAKTKPREEQFNSTFRVVSVLTVVHQDWLNGKEYKCKVSNKGLPA PIEKTISKTK |
| Amino acid sequence corresponding to the residues 233rd to 332nd of Fc re gion of IgG2 antibody according to Eu numbering (SEQ ID No: 20): |
| PVAGPSVFLFPPKPKDTLMISRTPEVTCVVVDVSHEDPEVQFNWYVDGVE VHNAKTKPREEQFNSTFRVVSVLTVVHQDWLNGKEYKCKVSNKGLPAPI |

### Construction of Fc-Mutant Library

Point mutation libraries are designed to introduce mutations through degenerate primers, and multiple degenerate primers are designed. For example, the degenerate primer used for the P233 point mutation is CCACCGTGCCCAGCACCANNSGTGGCAGGACCGTCAGTC (SEQ ID NO: 21). The mutant library fragment and the connection vector are obtained by PCR, and ligated by Multi One Step Cloning Kit (Yeasen Biotechnology (Shanghai) Co., Ltd., China) and transformed into DH5α to obtain a mutant library. For each library, 20 colonies were picked for sequencing to confirm whether a mutation was introduced at the target location.

The method for the combined mutation library is similar. The primers are designed according to the mutation type with enrichment which has been screened according to the point mutation library. The intermediate degenerate primer part is modified to be a primer sequence comprising a codon encoding a specific amino acid or a degenerate codon encoding several amino acids, for example, L328W (TGCAAGGTCTCCAACAAAGGCTGGCCAGCCCCCATCGAGAAAAC, SEQ ID NO: 22), Combine2_1 (ACGTGCGTGGTGGTGGACKNGWBGVASRWSSACCHGGAGGTCCAGTTC AACTGG, SEQ ID NO: 23).

In addition to the normal base, R in the above primers represents A/G, Y represents C/T, M represents A/C, K represents G/T, S represents G/C, W represents A/T, H represents A/T/C, B represents G/T/C, V represents G/A/C, D represents G/A/T, and N represents A/T/C/G.

### Construction of stably-expressing cell lines

The library plasmid and the helper plasmid Phoenix-Eco (molar ratio 2:1) were mixed with transfection reagent PEI (mass ratio 1:5) and added into plated Phoenix cells (confluence 80%), changing fresh culture after 6 hours, and the supernatant containing retrovirus was collected after 48 hours. Plated 3T3 cells with a confluence of 80% prepared in advance were infected by 50% fresh medium plus 50% of the above retrovirus supernatant. The solution was changed after 24 hours. After 48 hours, the cell line stably expressing the antibody can be obtained.

### Flow cytometry analysis of the cell lines stably expressing the antibody

3T3 cells were collected by trypsin digestion, and a single cell suspension was prepared. About 1-5^{∗}10⁶ cells were re-suspended in 50µl FACS buffer (1xPBS containing 0.5% FBS and 2mM EDTA) supplied with PE-F(ab')2 Fragment Goat Anti-Human IgG (1:500, Jackson ImmunoResearch Laboratories) or Biotin-FcyRIIB (1µg/ml, Sino Biological) and Streptavidin-APC (1:500, BD Biosciences). The cells were incubated for 15 minutes on ice and then washed twice with FACS buffer and re-suspended in 200 µl FACS buffer and analyzed by flow cytometry.

### Sorting of cells with high affinity to FcyRIIB by Flow cytometry

The staining system is amplified according to the above-mentioned flow cytometry analysis and staining method, and the cell is re-suspended to the final concentration of ~ 1-5^{∗}10⁷ cells/ml before loading. Cells expressing IgG (strong PE fluorescence signal) and having high affinity to FcyRIIB (strong APC fluorescence signal) are sorted, and the proportion is controlled to be less than 1% of IgG positive cells.

### Magnetic-activated sorting of cells with high affinity to FcyRIIB and low affinity to activating FcyR (high I/A ratio)

3T3 cells were collected by trypsin digestion, and a single cell suspension was prepared. About 1^{∗}10⁷ cells were re-suspended in 50µl MACS buffer (1xPBS containing 0.5% BSA and 2mM EDTA) supplied with Biotin-FcyRIIB (1µg/ml, Sino Biological) and activating FcyRs (including FcyRI, FcγRIIA^{131H}, FcγRIIA^{131R}, FcγRIIIA^{158V}, FcγRIIIA^{158F}, all for 1µg/ml, Shanghai NOVOPROTEIN SCIENTIFIC INC.). The cells were incubated for 15 minutes on ice and then washed twice with MACS buffer and re-suspended in 100 µl MACS buffer. Twenty-five µl anti-Biotin magnetic beads were added, and the cells were incubated for 15 minutes on ice and then washed once with MACS buffer and re-suspended to 500 µl, and added to the LS column put in the magnetic field. The effluent was collected, washed with 3 mL of MACS buffer and collected; The LS column was then removed out of the magnetic field, and 5 mL of MACS buffer was used to rapidly elute the remaining cells from the sorting column to collect positive cells, which are the high I/A ratio cells.

### High throughput sequencing analysis

About 0.1-5^{∗}10⁶ cells of the target cell population were taken and subject to cell lysis genomic DNA extraction, PCR (amplification of Fc fragment). Library construction and high-throughput sequencing (PE 150 or PE 250) were performed, and about 60,000-300,000 sequences were obtained for each sample. The nucleotide sequences of the mutation region were extracted and translated into the corresponding amino acid sequences (http://www.cbs.dtu.dk/services/VirtualRibosome/, Rasmus Wernersson. Nucl. Acids Res. 2006 34: W385-W388). The proportion of each mutation sequence in each sample was calculated, wherein: the proportion of the mutation sequence = the mutation sequence count/the total sequence count.

### Antibody expression and purification

An advantageous Fc mutant or a new combined mutant according to NGS is constructed into the heavy chain of the anti-human OX40 antibody, which is transiently transfected into HEK293S cells together with the corresponding light chain to express proteins. Protein G purification is performed to obtain different Fc mutant antibodies, and SDS-PAGE is used to confirm that all the antibody structures are intact. The anti-human OX40 antibody DNA sequence and protein sequence are shown in Table 5 and Table 6.

**Table 5. DNA sequence of wild-type IgG2 anti-human OX40 antibody**

| |
|---|
| Antibody light chain DNA sequence (SEQ ID NO:16) |
| ATGGAAACCCCAGCGCAGCTTCTCTTCCTCCTGCTACTCTGGCTCCCA GATACCACCGGAGAAATTGTGTTGACGCAGTCTCCAGGCACCCTGTC TTTGTCTCCAGGGGAAAGAGCCACCCTCTCCTGCAGGGCCAGTCAGA GTGTTAGCGGCGACTACTTAGCCTGGTACCAGCAGAAACTTGGCCAG |
| GCTCCCAGGCTCCTCATCTATGGTGCATCCATCAGGGCCACTGGCATC CCAGACAGGTTCAGTGGCAGTGGGTCTGGGACAGACTTCACTCTCAC CATCAGCAAACTGGAGCCTGAAGATTTTGCAGTGTATTACTGTCAGC AGTATGGTAGCTCACCGCTCACTTTCGGCGGAGGGACCAAGGTGGAG ATCAAACGAACTGTGGCTGCACCATCTGTCTTCATCTTCCCGCCATCT GATGAGCAGTTGAAATCTGGAACTGCCTCTGTTGTGTGCCTGCTGAAT AACTTCTATCCCAGAGAGGCCAAAGTACAGTGGAAGGTGGATAACGC CCTCCAATCGGGTAACTCCCAGGAGAGTGTCACAGAGCAGGACAGCA AGGACAGCACCTACAGCCTCAGCAGCACCCTGACGCTGAGCAAAGCA GACTACGAGAAACACAAAGTCTACGCCTGCGAAGTCACCCATCAGGG CCTGAGCTCGCCCGTCACAAAGAGCTTCAACAGGGGAGAGTGTTGA |
| Wild-type IgG2 antibody heavy chain DNA sequence (SEQ ID NO:17) |
| ATGAAACACCTGTGGTTCTTCCTCCTCCTGGTGGCAGCTCCCAGATGG GTCCTGTCTCAGGTGCAGCTGCAGGAGTCGGGCCCAGGACTGGTGAA GCCTTCGGGGACCCTGTCACTCACCTGCGCTGTCTCTGGTGGCTCCAT CAGCAGTAGTATCTGGTGGAGCTGGGTCCGCCAGCCCCCAGGGAAGG GGCTGGAATGGATTGGGGAAATCCATCATAGTGGGAACACCAACTGC AATCCGTCCCTCAAGAGTCGAGTCACCATATCAATAGACAAGTCCAA GAACCAGTTCTCCCTGAAACTGACCTCTGTGACCGCCGCGGACACGG CCGTATATTATTGTGCGAGAGCGGGAACTGGAACTACGCTTGACTAC TGGGGCCAGGGAACCCTGGTCACCGTCTCGAGCGCCTCCACCAAGGG CCCATCGGTCTTCCCCCTGGCGCCCTGCTCCAGGAGCACCTCCGAGAG CACAGCGGCCCTGGGCTGCCTGGTCAAGGACTACTTCCCCGAACCGG TGACGGTGTCGTGGAACTCAGGCGCTCTGACCAGCGGCGTGCACACC TTCCCAGCTGTCCTACAGTCCTCAGGACTCTACTCCCTCAGCAGCGTG GTGACCGTGCCCTCCAGCAACTTCGGCACCCAGACCTACACCTGCAA CGTAGATCACAAGCCCAGCAACACCAAGGTGGACAAGACAGTTGAG CGCAAATGTTGTGTCGAGTGCCCACCGTGCCCAGCACCACCTGTGGC AGGACCGTCAGTCTTCCTCTTCCCCCCAAAACCCAAGGACACCCTCAT GATCTCCCGGACCCCTGAGGTCACGTGCGTGGTGGTGGACGTGAGCC ACGAAGACCCCGAGGTCCAGTTCAACTGGTACGTGGACGGCGTGGAG GTGCATAATGCCAAGACAAAGCCACGGGAGGAGCAGTTCAACAGCA CGTTCCGTGTGGTCAGCGTCCTCACCGTTGTGCACCAGGACTGGCTGA ACGGCAAGGAGTACAAGTGCAAGGTCTCCAACAAAGGCCTCCCAGCC CCCATCGAGAAAACCATCTCCAAAACCAAAGGGCAGCCCCGAGAACC ACAGGTGTACACCCTGCCCCCATCCCGGGAGGAGATGACCAAGAACC AGGTCAGCCTGACCTGCCTGGTCAAAGGCTTCTACCCCAGCGACATC GCCGTGGAGTGGGAGAGCAATGGGCAGCCGGAGAACAACTACAAGA CCACACCTCCCATGCTGGACTCCGACGGCTCCTTCTTCCTCTACAGCA AGCTCACCGTGGACAAGAGCAGGTGGCAGCAGGGGAACGTCTTCTCA TGCTCCGTGATGCATGAGGCTCTGCACAACCACTACACGCAGAAGAG CCTCTCCCTGTCTCCGGGTAAATGA |

**Table 6. Protein sequence of wild-type IgG2 anti-human OX40 antibody**

| |
|---|
| Antibody light chain protein sequence (SEQ ID NO:18) |
| METPAQLLFLLLLWLPDTTGEIVLTQSPGTLSLSPGERATLSCRASQSVSG DYLAWYQQKLGQAPRLLIYGASIRATGIPDRFSGSGSGTDFTLTISKLEPE DFAVYYCQQYGSSPLTFGGGTKVEIKRTVAAPSVFIFPPSDEQLKSGTAS VVCLLNNFYPREAKVQWKVDNALQSGNSQESVTEQDSKDSTYSLSSTLT LSKADYEKHKVYACEVTHQGLSSPVTKSFNRGEC |
| Wild-type IgG2 antibody heavy chain protein sequence (SEQ ID NO:19) |
| MKHLWFFLLLVAAPRWVLSQVQLQESGPGLVKPSGTLSLTCAVSGGSIS SSIWWSWVRQPPGKGLEWIGEIHHSGNTNCNPSLKSRVTISIDKSKNQFS LKLTSVTAADTAVYYCARAGTGTTLDYWGQGTLVTVSSASTKGPSVFP LAPCSRSTSESTAALGCLVKDYFPEPVTVSWNSGALTSGVHTFPAVLQSS GLYSLSSVVTVPSSNFGTQTYTCNVDHKPSNTKVDKTVERKCCVECPPC PAPPVAGPSVFLFPPKPKDTLMISRTPEVTCVVVDVSHEDPEVQFNWYV DGVEVHNAKTKPREEQFNSTFRVVSVLTVVHQDWLNGKEYKCKVSNK GLPAPIEKTISKTKGQPREPQVYTLPPSREEMTKNQVSLTCLVKGFYPSDI AVEWESNGQPENNYKTTPPMLDSDGSFFLYSKLTVDKSRWQQGNVFSC SVMHEALHNHYTQKSLSLSPGK |

### The analysis of the binding properties of the mutant antibody to FcyR by Enzyme-linked immunosorbent assay (ELISA)

One hundred µl (2 µg/mL) of antibody or mutant thereof were added to the enzyme-linked plate to be coated overnight, then the supernatant was discarded. The plate was blocked with PBS containing 1% BSA for 2 hours, and washed with PBST (PBS containing 0.05% Tween 20). Biotin-labeled FcyR extracellular domain protein (Beijing Sino Biological Co., Ltd) of appropriate concentration was added and incubated at room temperature for 1 hour, then the supernatant was discarded and the plate was washed with PBST. The Biotin-labeled protein was detected by incubating with Streptavidin-HRP (BD BioSciences) at room temperature for 1 hour. The supernatant was removed, and the color-developing solution was added for color development for 20-40 minutes to determine the absorbance of 650 nm (A650).

### Immune activation activity analysis (promoting T cell proliferation) of anti-human OX40 antibody with different antibody Fc mutant in vitro

Spleen was isolated from humanized FcyR/OX40 mouse to prepare a single cell suspension after lysing red blood cells. CFSE labeling was performed. Cells were re-suspended in PBS containing 5 µM CFSE to a concentration of 5^{∗}10⁶/mL and incubated at 37°C for 15 minutes. Cells were then washed twice with 5% FBS-containing PBS and spinned down at 600g for 5 min, followed by re-suspending the cells with primary cell culture medium (RPMI + 10% FBS + 1% Pen/Strep +1% HEPES +1% Sodium Pyruvate + 0.1% 2ME (final 50µM) + 1% L-glutamine + 1% Non-essential A.A.) to a concentration of 3^{∗}10⁶/ml. Each well was added with 100 µl of cell suspension, i.e., 3^{∗}10⁵ cells per well. The (anti-hOX40) antibody concentrations were diluted to 2, 0.2 and 0.02 µg/ml, respectively, using the primary cell culture medium containing 0.2 µg/ml anti-CD3. One hundred µl of the prepared antibody mixture was transferred into the wells containing CFSE-labeled humanized FCyR/OX40 spleen cells. Two groups of controls were: CD3 only groups containing CFSE-labeled cells and anti-mouse CD3, but without any other antibodies; and CD3 + CD28 groups containing CFSE-labeled cells and anti-mouse CD3 and 2 µg/mL of anti-mouse CD28 antibody (Clone 37.51 (RUO), BD Biosciences) (the final concentrations of anti-mouse CD3 and CD28 were 0.1µg/ml and 1µg/ml, respectively). The cells were incubated at 37°C for 3 days in a 5% CO₂ thermostatic cell incubator. After the culturing was finished, the cells were collected and analyzed for the number and proliferation of CD4⁺ and CD8⁺ cells by flow cytometry. Proliferation of T cells analyzed by flow cytometry: the cultured cells were transferred to a 96-well U bottom plate, washed twice with PBS, centrifuged at 500g for 5 minutes, then the supernatant was discarded. The cells were re-suspended in 50 µl of FACS buffer (PBS containing 0.5% FBS, 2mM EDTA) containing PE anti-Mouse CD4 (Clone: GK1.5, 1:500, BD) and APC anti-Mouse CD8a (Clone: 53-6.7, 1:500, BioLegend), incubated on ice in dark for 15 minutes, then washed twice with PBS buffer and re-suspended in 200 µl of FACS buffer containing DAPI (0.5 µg/ml, Invitrogen) and CountBright Absolute Counting Beads (Life Technologies, 2µl/sample), and analyzed by flow cytometry.

The present invention mainly provides antibody Fc mutant sequences that enhance human IgG2 interaction with FcγRIIB. Meanwhile, the present invention also provides a method for screening and obtaining these mutants. As shown in Figure 2, Fc mutants with specific affinity properties can be screened according to the above method.

Furthermore, those skilled in the art can screen amino acid mutations that enhance various protein-protein interactions or protein-other molecules interactions according to the above screening method provided by the present invention; a general screening method comprises:
1) providing a parent protein sequence, introducing amino acid mutations in the parent protein sequence by PCR;
2) constructing the parent protein and the mutated protein into an expression vector to form a mutation library;
3) transfecting the expression vector into mammalian cells, and expressing the parent protein and the mutated protein on the cell surface;
4) incubating a labeled interacting protein and the mammalian cell, and selecting labeled mammalian cells by flow cytometry or magnetic-activated cell sorting;
5) extracting DNAs from the labeled mammalian cells, sequencing the DNAs, and analyzing the change of the sequence proportions before and after sorting; wherein, the amino acid mutation in which the proportion after sorting is significantly higher than the proportion before sorting is an amino acid mutation capable of enhancing the binding affinity between the protein and the interacting protein.

### Example 1: Construction of Human Antibody IgG2_Fc Point Mutation Library

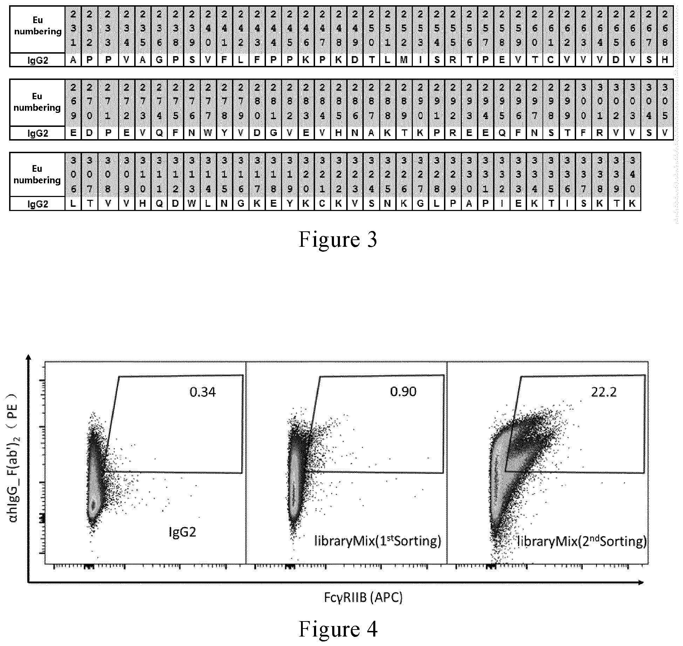

Some sites on human IgG2_Fc_CH2 are selected, including P233-S239, V266-P271, S298-T299 and G327-I332 (Eu numbering, see FIGS. 3 and 4). Amino acids on these sites are randomly mutated, and each site comprises 20 types of amino acids (A, R, D, C, Q, E, H, I, G, N, L, K, M, F, P, S, T, W, Y, V) in total, including wild-type. Single-point random mutation plasmid library corresponding to the above four regions of human IgG2_Fc respectively (Library 1, Library2, Library3, Library4), and a mixture library of these four libraries (LibraryMix) were constructed.

### Example 2: Cell enrichment after flow cytometry sorting relative to before sorting

After the plasmid library is successfully constructed, retrovirus is prepared to infect 3T3 cells, to obtain the cell lines stably expressing the libraries. The present invention uses flow cytometry sorting techniques to sort cells expressing human IgG2 (detected by anti-human IgG F(ab')₂) and binding human FcyRIIB. Figure 4 shows that flow cytometry sorting can gradually enrich cells with high binding affinity to FcyRIIB. The cells obtained by flow cytometry sorting for different rounds of human IgG2 library LibraryMix are respectively analyzed. The flow cytometry analysis shows that the proportion of the cells binding to FcyRIIB is increased after each round. The proportion after the first round is 0.90% and that of the second round is 22.2%, which increased 2.65 times and 65.29 times, respectively, as compared to 0.34% of wild-type human IgG2.

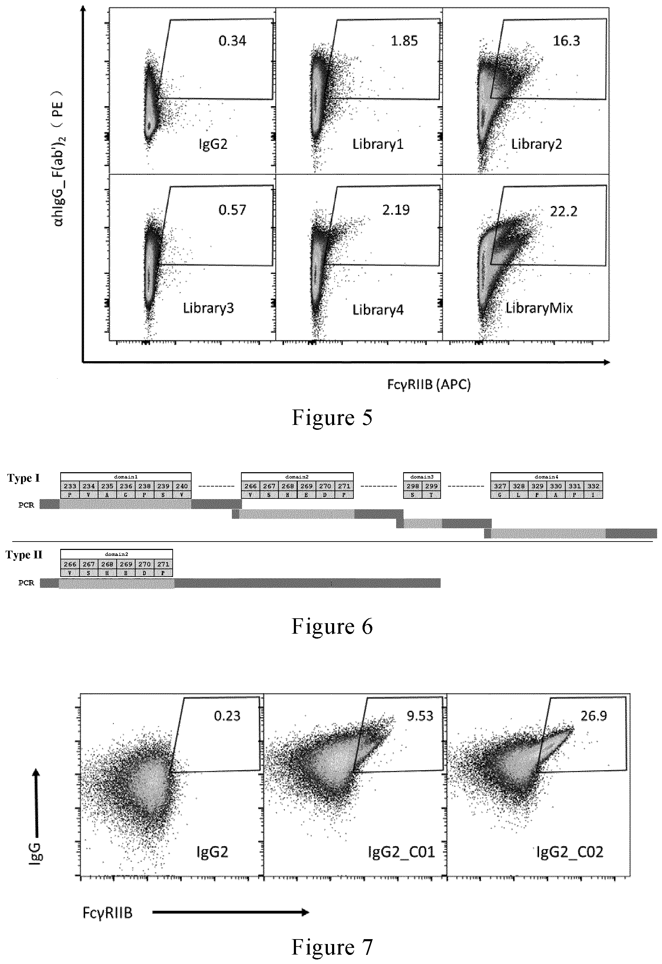

As shown in FIG. 5, after two rounds of sorting, the proportion of the FcyRIIB-binding cells of Library1, Library2, Library3, Library4 and LibraryMix detected by flow cytometry are increased to 1.85%, 16.3%, 0.57%, 2.19% and 22.2%, respectively, which are 5.44 times, 47.94 times, 1.69 times, 6.44 times and 65.29 times higher than 0.34% of wild type human IgG2.

### Example 3: NGS analysis of IgG2 Fc mutation types with enhanced FcyRIIB binding affinity after flow cytometry sorting.

Mutations with the ratio of the proportion of sequence after sorting to the proportion before sorting increased to be greater than twice that of wild-type human IgG2 (enrichment factor/wild-type enrichment factor > 2) are selected, i.e., mutations on human IgG2 which is beneficial to the improvement of the binding affinity of IgG2 to FcyRIIB (at least twice that of the wild-type enrichment factor), see Table 7. For example, the proportion of L328W before sorting is 0.39%, and the proportion after sorting is 12.84%, therefore the enrichment factor is 33.20, which is 22.21 times the wild-type enrichment factor (1.49).

**Table 7. Mutations in human IgG2 which is beneficial to the improvement of the binding affinity of IgG2 to FcyRIIB**

| **NO.** | **Mutation** | **Before sorting** | | **After sorting** | | **Enrichmen t factor** | **Increased factor** |
|---|---|---|---|---|---|---|---|
| | | **count** | **Percen tage** | **Count** | **Perce ntage** | **Percentage after sort ing/percent age before sorting** | **Enrichme nt factor/ wild-type enrichm ent factor** |
| Library2 | | | | | | | |
| #1 | Wild-type | 12137 | 11.34 | 7266 | 6.51 | 0.57 | 1.00 |
| 1 | S267Q | 410 | 0.38 | 3693 | 3.31 | 8.64 | 15.05 |
| 2 | S267A | 744 | 0.70 | 2457 | 2.20 | 3.17 | 5.52 |
| 3 | S267D | 513 | 0.48 | 1128 | 1.01 | 2.11 | 3.67 |
| 4 | S267M | 794 | 0.74 | 1223 | 1.10 | 1.48 | 2.57 |
| 5 | S267G | 10 | 0.00 | 27 | 0.01 | 1.79 | 3.50 |
| 6 | H268E | 319 | 0.30 | 561 | 0.50 | 1.69 | 2.94 |
| 7 | H268D | 221 | 0.21 | 355 | 0.32 | 1.54 | 2.68 |
| 8 | H268K | 421 | 0.39 | 581 | 0.52 | 1.32 | 2.31 |
| 9 | P271C | 893 | 0.83 | 15969 | 14.30 | 17.14 | 29.87 |
| 10 | V266A/P271C | 5 | 0.00 | 220 | 0.20 | 42.18 | 73.50 |
| 11 | V266A/P271G | 3 | 0.00 | 27 | 0.02 | 8.63 | 15.03 |
| 12 | S267A/P271C | 3 | 0.00 | 117 | 0.10 | 37.39 | 65.14 |
| 13 | S267A/P271G | 2 | 0.00 | 11 | 0.01 | 5.27 | 9.19 |
| 14 | S267E/D270H | 2 | 0.00 | 89 | 0.08 | 42.66 | 74.33 |
| 15 | S267E/P271C | 2 | 0.00 | 2089 | 1.87 | 1001.34 | 1744.71 |
| 16 | S267E/P271V | 2 | 0.00 | 46 | 0.04 | 22.05 | 38.42 |
| 17 | S267E/P271W | 2 | 0.00 | 50 | 0.04 | 23.97 | 41.76 |
| 18 | S267E/P271Y | 4 | 0.00 | 80 | 0.07 | 19.17 | 33.41 |
| 19 | S267M/P271C | 2 | 0.00 | 67 | 0.06 | 32.12 | 55.96 |
| 20 | S267M/P271G | 2 | 0.00 | 9 | 0.01 | 4.31 | 7.52 |
| 21 | V266G/P271C | 3 | 0.00 | 12 | 0.01 | 3.83 | 6.68 |

| Library4 | | | | | | | |
|---|---|---|---|---|---|---|---|
| #2 | wild-type | 14727 | 16.91 | 17343 | 25.27 | 1.49 | 1.00 |
| 22 | G327A | 713 | 0.82 | 2838 | 4.13 | 5.05 | 3.38 |
| 23 | L328F | 3 | 0.00 | 1332 | 1.94 | 563.47 | 377.03 |
| 24 | L328W | 337 | 0.39 | 8815 | 12.84 | 33.20 | 22.21 |
| 25 | L328Y | 50 | 0.06 | 133 | 0.19 | 3.38 | 2.26 |
| 26 | G327A/A330R | 2 | 0.00 | 155 | 0.23 | 98.35 | 65.81 |
| 27 | G327A/A330V | 2 | 0.00 | 11 | 0.02 | 6.98 | 4.67 |
| 28 | G327A/I332A | 2 | 0.00 | 46 | 0.07 | 29.19 | 19.53 |
| 29 | G327A/I332C | 1 | 0.00 | 9 | 0.01 | 11.42 | 7.64 |
| 30 | G327A/I332E | 1 | 0.00 | 99 | 0.14 | 125.64 | 84.07 |
| 31 | L328E/I332T | 10 | 0.01 | 434 | 0.63 | 55.08 | 36.85 |

| LibraryMix | | | | | | | |
|---|---|---|---|---|---|---|---|
| #3 | wild-type | 91423 | 29.03 | 119849 | 21.72 | 0.75 | 1.00 |
| 32 | S267E | 259 | 0.08 | 98580 | 17.86 | 217.23 | 290.34 |
| 33 | S267R | 6 | 0.00 | 16 | 0.00 | 1.52 | 2.03 |
| 34 | P271G | 1132 | 0.36 | 136659 | 24.77 | 68.90 | 92.09 |
| 35 | P271Q | 218 | 0.07 | 631 | 0.11 | 1.65 | 2.21 |
| 36 | S298L | 2696 | 0.86 | 10298 | 1.87 | 2.18 | 2.91 |
| 37 | L328M | 14 | 0.00 | 611 | 0.11 | 24.91 | 33.29 |
| 38 | S267V/S298L | 9 | 0.00 | 33 | 0.01 | 2.09 | 2.80 |
| 39 | S267E/S298R | 7 | 0.00 | 402 | 0.07 | 32.78 | 43.81 |
| 40 | P271A/S298R | 7 | 0.00 | 30 | 0.01 | 2.45 | 3.27 |
| 41 | P271G/G236V | 6 | 0.00 | 108 | 0.02 | 10.27 | 13.73 |
| 42 | P271G/P329S | 7 | 0.00 | 567 | 0.10 | 46.23 | 61.79 |
| 43 | P271G/P331C | 7 | 0.00 | 1717 | 0.31 | 139.99 | 187.11 |
| 44 | P271G/P331T | 12 | 0.00 | 680 | 0.12 | 32.34 | 43.23 |
| 45 | P271G/S298D | 8 | 0.00 | 95 | 0.02 | 6.78 | 9.06 |
| 46 | P271G/S298E | 10 | 0.00 | 5218 | 0.95 | 297.81 | 398.04 |
| 47 | P271G/S298G | 20 | 0.01 | 341 | 0.06 | 9.73 | 13.01 |
| 48 | P271G/S298K | 6 | 0.00 | 107 | 0.02 | 10.18 | 13.60 |
| 49 | P271G/S298L | 13 | 0.00 | 4286 | 0.78 | 188.17 | 251.50 |
| 50 | P271G/S298N | 8 | 0.00 | 252 | 0.05 | 17.98 | 24.03 |
| 51 | P271G/S298R | 18 | 0.01 | 504 | 0.09 | 15.98 | 21.36 |
| 52 | P271G/T299A | 23 | 0.01 | 253 | 0.05 | 6.28 | 8.39 |
| 53 | P271G/T299M | 10 | 0.00 | 83 | 0.02 | 4.74 | 6.33 |
| 54 | P271G/T299S | 6 | 0.00 | 126 | 0.02 | 11.99 | 16.02 |
| 55 | P271G/T299W | 6 | 0.00 | 97 | 0.02 | 9.23 | 12.33 |

| | | | | | | | |
|---|---|---|---|---|---|---|---|
| # 1-3: Data from the experiments of Library 2 (#1), Library 4 (#2), and LibraryMix (#3). | | | | | | | |

In addition to single point mutation, some double mutations were also obtained, wherein the ratios of these amino acid mutations after flow cytometry sorting to that before sorting are increased by more than twice as compared to the wild-type human IgG2. The double mutation combination (V266A/P271C or G, S267A/P271C or G, S267E/D270H, S267E/P271C or V or W or Y, S267E/S298R, S267M/P271C or G, P271A/S298R, P271G/G236V, P271G/G329S, P271G/P331C or T, P271G/S298D or E or G or K or L or N or R, P271G/T299A or M or S or W, G327A/A330R or V, G327A/I332A or C or E, L328E/I332T, etc.) has a higher increased factor (ratio) than any point mutation thereof, indicating that both amino acids in the mutation combination contribute to the increase in the binding affinity to FcyRIIB. In addition, mutation combinations such as S267V/S298L provide an increased factor approximately equal to S298L mutation, which has a higher increased factor over S267V, indicating that the improvement of the mutation combination is basically only the contribution of S298L, rather than the other point mutation, as shown in Table 8. Therefore, in addition to the amino acid mutation provided by the present invention, the Fc region, the antibody, the Fc fragment or the fusion protein of the present invention may further comprise other possible combinations or modifications.

**Table 8. Double mutations on human IgG2 which is beneficial to the improvement of the binding affinity of IgG2 to FcγRIIB**

| **NO.** | **Mutation** | **Enrichmen t factor** | **Increased factor ^{a}** | **Increased factor b** | | **Increased factor 2 ^{c}** | |
|---|---|---|---|---|---|---|---|
| | | **Percentage after sort ing/percen tage before sorting** | **Enrichmen t factor/wi ld-type en richment factor** | **Site 1** | **Site 2** | **Site 1** | **Site 2** |
| 1 | V266A/P271C | 42.18 | 73.5 | 3.1 | 29.9 | 24.1 | 2.5 |
| 2 | V266A/P271G | 8.63 | 15.03 | 3.1 | 4.7 | 4.9 | 3.2 |
| 3 | S267A/P271C | 37.39 | 65.14 | 5.5 | 29.9 | 11.8 | 2.2 |
| 4 | S267A/P271G | 5.27 | 9.19 | 5.5 | 4.7 | 1.7 | 1.9 |
| 5 | S267E/D270H | 42.66 | 74.33 | 28.5 | 1.3 | 2.6 | 56.7 |
| 6 | S267E/P271C | 1001.34 | 1744.71 | 28.5 | 29.9 | 61.2 | 58.4 |
| 7 | S267E/P271V | 22.05 | 38.42 | 28.5 | 0.7 | 1.4 | 52.6 |
| 8 | S267E/P271W | 23.97 | 41.76 | 28.5 | 0.4 | 1.5 | 101.9 |
| 9 | S267E/P271Y | 19.17 | 33.41 | 28.5 | 1.0 | 1.2 | 33.4 |
| 10 | S267E/S298R | 32.78 | 43.81 | 28.5 | 0.8 | 1.5 | 56.2 |
| 12 | S267M/P271C | 32.12 | 55.96 | 2.6 | 29.9 | 21.8 | 1.9 |
| 13 | S267M/P271G | 4.31 | 7.52 | 2.6 | 4.7 | 2.9 | 1.6 |
| 14 | P271A/S298R | 2.45 | 3.27 | 0.1 | 0.8 | 54.5 | 4.2 |
| 15 | P271G/G236V | 10.27 | 13.73 | 4.7 | 0.5 | 2.9 | 30.5 |
| 16 | P271G/P329S | 46.23 | 61.79 | 4.7 | 1.2 | 13.1 | 52.8 |
| 17 | P271G/P331C | 139.99 | 187.11 | 4.7 | 0.2 | 39.6 | 779.6 |
| 18 | P271G/P331T | 32.34 | 43.23 | 4.7 | 0.1 | 9.1 | 480.3 |
| 19 | P271G/S298D | 6.78 | 9.06 | 4.7 | 1.6 | 1.9 | 5.5 |
| 20 | P271G/S298E | 297.81 | 398.04 | 4.7 | 7.1 | 84.2 | 56.0 |
| 21 | P271G/S298G | 9.73 | 13.01 | 4.7 | 1.2 | 2.8 | 10.7 |
| 22 | P271G/S298K | 10.18 | 13.6 | 4.7 | 4.4 | 2.9 | 3.1 |
| 23 | P271G/S298L | 188.17 | 251.5 | 4.7 | 2.9 | 53.2 | 86.4 |
| 24 | P271G/S298N | 17.98 | 24.03 | 4.7 | 2.3 | 5.1 | 10.5 |
| 25 | P271G/S298R | 15.98 | 21.36 | 4.7 | 0.8 | 4.5 | 27.4 |
| 26 | P271G/T299A | 6.28 | 8.39 | 4.7 | 0.4 | 1.8 | 20.5 |
| 27 | P271G/T299 M | 4.74 | 6.33 | 4.7 | 1.0 | 1.3 | 6.1 |
| 28 | P271G/T299S | 11.99 | 16.02 | 4.7 | 0.6 | 3.4 | 25.0 |
| 29 | P271G/T299 W | 9.23 | 12.33 | 4.7 | 0.5 | 2.6 | 23.3 |
| 30 | G327A/A330 R | 98.35 | 65.81 | 3.4 | 0.0 | 19.5 | 21936.7 |
| 31 | G327A/A330 V | 6.98 | 4.67 | 3.4 | 1.0 | 1.4 | 4.8 |
| 32 | G327A/I332A | 29.19 | 19.53 | 3.4 | 16.4 | 5.8 | 1.2 |
| 33 | G327A/I332C | 11.42 | 7.64 | 3.4 | 2.7 | 2.3 | 2.9 |
| 34 | G327A/I332E | 125.64 | 84.07 | 3.4 | 1.1 | 24.9 | 79.3 |
| 35 | L328E/I332T | 55.08 | 36.85 | 0.1 | 1.6 | 335.0 | 22.6 |

| | | | | | | | |
|---|---|---|---|---|---|---|---|
| a. Ratio of mutant enrichment factor/wild-type enrichment factor after sorting. b. Ratio of mutant enrichment factor/wild-type enrichment factor after sorting regarding the original point mutation. c. Ratio of increase factor of combined mutation/increase factor of one point mutation. | | | | | | | |

### Example 4: Analysis of favorable mutant types by NGS after magnetic-activated sorting

The high-FcyRIIB-affinity cells obtained from the above flow cytometry sorting are further sorted by **magnetic-activated** sorting. The cells are incubated with the mixture of Biotin-labeled FcyRIIB and non-labeled activating FcyRs, and then the labeled cells are sorted by anti-Biotin magnetic beads. The enrichment ratios after sorting relative to before sorting are analyzed by NGS. Mutations with a higher I/A ratio can be screened, as shown in Table 9. Mutations are selected according to the following criteria: the ratio of the mutation after sorting to that before sorting is increased by more than twice as compared to the wild-type human IgG2, and the proportion of the mutation is decreased in the sorting portion not binding to magnetic bead (i.e., the outflow liquid passing the LS column).

**Table 9. Mutations in human IgG2 leading to the improvement of the binding affinity of human IgG2 to FcyRIIB, and showing lower binding affinity of human IgG2 to the activating FcyRs than that to FcyRIIB**

| **NO.** | **Muta tion** | **Before sorting** | | **Portion not bin ding to magneti c bead** | | **After sorting** | | **Comparison bef ore and after s orting** | | **Enric hmen t fact or** | **Incre ased factor** |
|---|---|---|---|---|---|---|---|---|---|---|---|
| | | **Coun t** | **Percen tage** | **Count** | **Percen tage** | **Amo unt** | **Percen tage** | **Percen tage a fter so rting/u nbindi ng per centag e** | **Percen tage b efore s orting/ unbind ing pe rcenta ge** | **Perce ntage after sorti ng/pe rcent age b efore sorti ng** | **Muta tion enric hmen t fact or/wil d-typ e enr ichme nt fa ctor** |
| Library2 | | | | | | | | | | | |
| #1 | Wild-type | 2838 | 3.60 | 5139 | 5.15 | 2509 | 2.98 | 0.58 | 0.70 | 0.83 | 1.00 |
| 1 | S267 N | 66 | 0.08 | NA | NA | 205 | 0.24 | - | - | 2.91 | 3.51 |
| 2 | S267 W | 89 | 0.11 | 4 | 0 | 177 | 0.21 | 52.49 | 28.16 | 1.86 | 2.25 |
| 3 | H268 N | 226 | 0.29 | 1 | 0 | 558 | 0.66 | 661.97 | 286.03 | 2.31 | 2.79 |
| 4 | P271 A | 61 | 0.08 | 3 | 0 | 180 | 0.21 | 71.18 | 25.73 | 2.77 | 3.33 |
| 5 | P271I | 311 | 0.39 | NA | NA | 873 | 1.04 | - | - | 2.63 | 3.17 |
| 6 | P271 L | 51 | 0.06 | 2 | 0 | 241 | 0.29 | 142.95 | 32.27 | 4.43 | 5.34 |
| 7 | P271 S | 64 | 0.08 | 10 | 0.01 | 187 | 0.22 | 22.18 | 8.1 | 2.74 | 3.3 |
| 8 | P271V | 246 | 0.31 | 1 | 0 | 923 | 1.1 | 1094.9 8 | 311.35 | 3.52 | 4.24 |

| Library4 | | | | | | | | | | | |
|---|---|---|---|---|---|---|---|---|---|---|---|
| #2 | Wild-type | 1193 7 | 14.33 | 16838 | 14.78 | 1355 4 | 12.62 | 0.85 | 0.97 | 0.88 | 1.00 |
| 9 | G327 L | 14 | 0.02 | 19 | 0.02 | 68 | 0.06 | 3.8 | 1.01 | 3.77 | 4.28 |
| 10 | G327 S | 83 | 0.1 | 11 | 0.01 | 738 | 0.69 | 71.16 | 10.32 | 6.9 | 7.84 |
| 11 | L328 N | 109 | 0.13 | 8 | 0.01 | 384 | 0.36 | 50.91 | 18.63 | 2.73 | 3.11 |

| Library Mix | | | | | | | | | | | |
|---|---|---|---|---|---|---|---|---|---|---|---|
| #3 | Wild-type | 1491 9 | 19.10 | 20509 | 20.31 | 2185 5 | 21.53 | 1.06 | 0.94 | 1.13 | 1.00 |
| 12 | L328 G | 12 | 0.02 | NA | NA | 47 | 0.05 | - | - | 3.01 | 2.67 |
| 13 | L328 R | 18 | 0.02 | 4 | 0 | 124 | 0.12 | 30.84 | 5.82 | 5.3 | 4.69 |
| 14 | L328 V | 17 | 0.02 | 2 | 0 | 51 | 0.05 | 25.37 | 10.99 | 2.31 | 2.04 |
| 15 | P271 G/S2 98N | 8 | 0.01 | 2 | 0 | 24 | 0.02 | 11.94 | 5.17 | 2.31 | 2.04 |
| 16 | H268 S | 30 | 0.04 | NA | NA | 306 | 0.3 | - | - | 7.85 | 6.95 |
| #4 | Wild-type | 1491 9 | 19.10 | 20860 | 18.89 | 1810 1 | 17.95 | 0.95 | 1.01 | 0.94 | 1.00 |
| 17 | P238 Q/S2 67E | 40 | 0.05 | 48 | 0.04 | 191 | 0.19 | 4.36 | 1.18 | 3.7 | 3.93 |
| 18 | S267 E/P32 9R | 27 | 0.03 | 30 | 0.03 | 169 | 0.17 | 6.17 | 1.27 | 4.85 | 5.16 |
| 19 | P271 G/I33 2L | 28 | 0.04 | 7 | 0.01 | 102 | 0.1 | 15.96 | 5.66 | 2.82 | 3 |
| 20 | S267 E | 13 | 0.02 | 1 | 0 | 48 | 0.05 | 52.56 | 18.38 | 2.86 | 3.04 |
| 21 | P271 G | 11 | 0.01 | 3 | 0 | 52 | 0.05 | 18.98 | 5.18 | 3.66 | 3.89 |
| 22 | L328 W | 18 | 0.02 | NA | NA | 99 | 0.1 | - | - | 4.26 | 4.53 |
| 23 | L328 A | 28 | 0.04 | NA | NA | 116 | 0.12 | - | - | 3.21 | 3.41 |
| 24 | L328 P | 29 | 0.04 | 3 | 0 | 81 | 0.08 | 29.57 | 13.67 | 2.16 | 2.3 |

| | | | | | | | | | | | |
|---|---|---|---|---|---|---|---|---|---|---|---|
| NA: In the sample of the column, the mutation of the row is not detected. #1-#4: Data from Library 2 (#1), Library 4 (#2), and two experiments of LibraryMix (#3, #4). | | | | | | | | | | | |

The affinity of human IgG1 to FcyRIIB can be increased by G236D, S239D (Seung Y Chu et al. Molecular Immunology 45 (2008) 3926-3933) and S298A mutation (US20090042291A1). However, in the results of NGS analysis of the present invention, the proportion of the IgG2 variant comprising G236D or S298A after sorting is not increased, but decreased (Table 10); the proportion of the human IgG2 variant containing S239D before sorting is 0.026%, and is not detected after sorting. These results demonstrate that G236D, S239D, and S298A mutations do not increase the FcyRIIB binding affinity of human IgG2, see Table 10.

**Table 10. The effect of the known mutation on the binding affinity of IgG2 to FcyR cannot be directly predicted according to the effect of the mutation on the binding affinity of IgG1 to FcyR.**

| **NO.** | **Mutant** | **Before sorting** | | **After sorting** | | **Enrichment facto r** |
|---|---|---|---|---|---|---|
| | | **Count** | **Percent age** | **Count** | **Percent age** | **Percentage after sorting/percentag e before sorting** |
| 1 | S298A | 4221 | 4.05 | 4205 | 3.72 | 0.92 |
| 2 | S239D | 29 | 0.03 | 0 | NA | - |
| 3 | G236D | 511 | 0.46 | 427 | 0.39 | 0.84 |

| | | | | | | |
|---|---|---|---|---|---|---|
| NA: The mutant of the row is not detected in the experiment of the column, and the percentage and factor cannot be calculated. | | | | | | |

### Example 5: Amino acid mutations in human IgG2 Fc polypeptide fragments which can enhance the affinity to FcyRIIB.

Table 11 summarizes the amino acid mutations capable of enhancing affinity to FcyRIIB screened by the point mutation library screening method according to the present invention. Compared with patents in the prior art, it has been found that the sites S267G and P271C of the present invention are both new mutation types on human IgG2 that can improve the binding affinity to FcyRIIB. The sites H268S, L328A and G327A are all new mutation types on human IgG2 that improve the binding affinity to FcyRIIB and show lower affinity to other activating FcyRs than that to FcγRIIB.

**Table 11. Amino acid mutations in human IgG2 that enhance the affinity to FcyRIIB**

| **Mutation site (Eu num bering)** | **Original amin o acid** | Amino acid mutation enhancing affinity to FcγRIIB | | | | | | | | | | |
|---|---|---|---|---|---|---|---|---|---|---|---|---|
| **267** | S | E | Q | A | D | M | G | R | V | N | W | |
| **268** | H | E | D | K | N | S | | | | | | |
| **271** | P | C | G | Q | V | w | Y | A | I | L | S | |
| **327** | G | A | L | S | | | | | | | | |
| **328** | L | F | M | W | Y | E | A | G | N | P | R | V |

### Example 6: Construction of Human Antibody IgG2_Fc Combined Mutation Library

According to the Fc mutation type capable of increasing the affinity to FcyRIIB (the mutation having a higher enrichment factor by more than 1 time over wild-type) screened by the above point mutation libraries and the high-throughput sequencing results, two types of combined mutation library were designed. Type I: combined point mutations among four regions, i.e., regions P233-V240, V266-P271, S298-T299 and G327-I332 each have 0-1 mutation, and the combined mutation contains 0-4 point mutations. Type II: combined mutation of multiple amino acids in one region, i.e., regions P233-V240, V266-P271, S298-T299 or G327-I332 has combined mutation within each region (respectively 0-7, 0-6, 0-2 and 0-6 combined mutations), as shown in FIG. 6. The size of the plasmid library is up to 4.67^{∗}10⁵.

### Example 7: Cell enrichment before and after flow cytometry sorting for combined mutation library

According to point mutation screening and high-throughput sequencing results, Type I and Type II combined mutation libraries are constructed (Example 6), and the cell libraries expressing the combined mutation are obtained. Then flow cytometry is used for sorting the cells with high affinity to FcyRIIB for two to three rounds. As shown in FIG. 7, after two rounds of sorting, the proportion of the cells of library IgG2_C01 and library IgG2_C02 detected by flow cytometry that are capable of binding to FcyRIIB are increased to 9.53% and 26.9%, respectively, which are 41.43 times and 116.96 times higher than 0.23% of wild type human IgG2. IgG2_C01 is a combined mutation (Type I) library having at most 1 mutation in each of the four regions of P233-V240, V266-P271, S298-T299, and G327-I332; IgG2_C02 is a cell library made by combining the combined mutation (Type II) libraries of four regions of P233-V240, V266-P271, S298-T299 and G327-I332 together and transferring it into 3T3 cells.

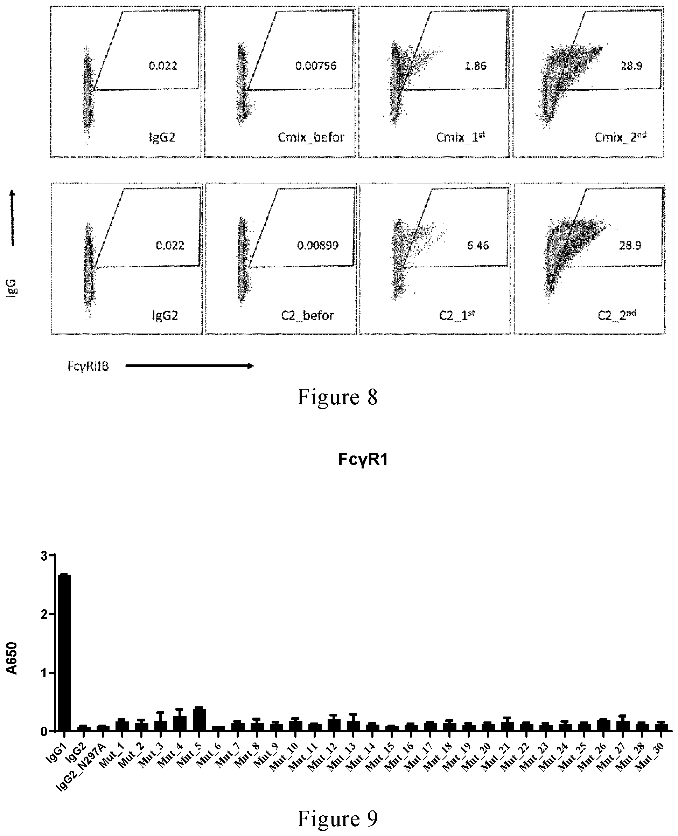

As shown in FIG. 8, after two rounds of sorting, the binding affinity of the cells of library Cmix and library C2 cells to FcyRIIB is increased significantly, according to flow cytometry. The proportion of the cells of the libraries that are capable of binding to FcyRIIB is increased upon each round of sorting: Cmix shows a cell proportion of 1.86% and 28.9% that are capable of binding to FcyRIIB after the first round of sorting and the second round of sorting, respectively, which are 246.03 times and 3822.75 times higher than 0.00756% before sorting; C2 reaches 6.46% and 28.9%, which are 718.58 times and 3214.68 times higher than 0.00899% before sorting. The library Cmix is a combined mutation (Type I) library with at most one mutation in each of the four regions; and the library C2 is a combined mutation (Type II) library of the V266-P271 region.

### Example 8: NGS analysis of IgG2 Fc combined mutation showing enhanced FcyRIIB binding affinity after flow cytometry sorting.

- In order to screen the IgG2 Fc combined mutation type with increased binding affinity to FcyRIIB, the combined mutant library constructed in the present invention is subjected to flow cytometry sorting to obtain cells with higher binding affinity to FcyRIIB, then the enrichment factor of each mutation type after sorting relative to before sorting is analyzed by NGS. The result is shown in Table 12. The screened mutation has a higher enrichment factor (after the last round of sorting/before sorting), or shows a significant enrichment in at least one round of sorting (after said round of sorting/before said round of sorting), or is only detected after the last round of sorting. For example, the enrichment factor of V266L/S298L/L328W is 21920.33 times of the wild type (after the last round of sorting/before sorting); the enrichment factor of the point mutation L328W is 8.78 times of the wild type (after the last round of sorting/before sorting); the enrichment factors of H268D/S298L/L328W are 43.98 (after the second round of sorting/before the second round of sorting) and 5.95 (after the third round of sorting/before the third round of sorting); the enrichment factor of V234M/S267E/S298L/L328W is 5.13 (after third round sorting/before third round sorting); V234Q/A235G/P238L/S239V/G327A/L328E/A330S/I332T is only detected after the last round of sorting.

**Table 12. Mutations on human IgG2 screened from the combined mutation library, which are beneficial to the improvement of the binding affinity of IgG2 to FcyRIIB**

| **N O.** | | **Percentage** | | | | **Enrichment factor** | | | | **Increased factor** |
|---|---|---|---|---|---|---|---|---|---|---|
| | | | **After 2nd sorti ng** | **After 1st s orting** | **Befor e sor ting** | | **Percentage after 2nd sorting/p ercentage after 1st s orting** | **Percentage after 1st sorting/per centage be fore sortin g** | **Percenta ge after 2nd sorti ng/perce ntage be fore sort ing** | **Mutant e nrichmen** t **factor/ wild-type enrichm ent facto r** |
| **#1** | **WT** | | **54.22** | **61.56** | **63.83** | | **0.88** | **0.96** | **0.85** | **1.00** |
| **1** | **L328W** | | **1.05** | **0.25** | **0.14** | | **4.26** | **1.75** | **7.46** | **8.78** |
| **2** | **S267E/A330S** | | **0.03** | **0.00** | **NA** | | **43.11** | **-** | **-** | **-** |
| **3** | **S267E/S298G** | | **0.25** | **0.01** | **0.00** | | **19.16** | **9.16** | **175.56** | **206.66** |
| **4** | **P233G/S267E** | | **0.05** | **0.01** | **0.00** | | **5.47** | **3.31** | **18.10** | **21.31** |
| **5** | **P233G/S267E/S 298G** | | **0.20** | **0.01** | **0.00** | | **38.49** | **7.13** | **274.31** | **322.90** |
| **#2** | **WT** | | **47.81** | **58.39** | **56.60** | | **0.82** | **1.03** | **0.84** | **1.00** |
| **6** | **V266L/S267E/H 268S/P271V** | | **0.25** | **NA** | **NA** | | **-** | - | - | - |
| **7** | **V234Q/A235G/ P238L/S239V/G 327A/L328E/A3 30S/I332T** | | **0.15** | **NA** | **NA** | | **-** | - | - | - |
| **8** | **V234Q/A235G/ P238L/S239V/S 267E/H268S/E2 69D** | | **0.07** | **NA** | **NA** | | **-** | - | - | - |
| **9** | **S267E/H268S/E 269D** | | **5.78** | **0.07** | **0.00** | | **83.65** | **80.49** | **6733.01** | **7971.36** |
| | | | | | | | | | | |

| | | **After 3rd sortin g** | **After 2nd sorti ng** | **After 1st s orting** | **Befor e sor ting** | **Percentag e after 3r d sorting/ percentage after 2nd sorting** | **Percentage after 2nd sorting/p ercentage after 1st s orting** | **Percentage after 1st sorting/per centage be fore sortin g** | **Percenta ge after 3rd sorti ng/perce ntage be fore sort ing** | **Mutant e nrichmen t factor/ wild-type enrichm ent facto r** |
|---|---|---|---|---|---|---|---|---|---|---|
| **#3** | **WT** | **0.40** | **1.03** | **1.32** | **1.07** | **0.39** | **0.78** | **1.24** | **0.38** | **1.00** |
| **10** | **H268D/S298L/L 328W** | **1.25** | **0.21** | **0.00** | **NA** | **5.95** | **43.98** | **-** | **-** | **-** |
| **11** | **V266L/L328W** | **1.20** | **0.22** | **0.00** | **0.00** | **5.53** | **53.28** | **4.71** | **1386.84** | **3695.56** |
| **12** | **V266L/S298L/L 328W** | **24.98** | **4.14** | **0.06** | **0.00** | **6.03** | **64.77** | **21.07** | **8226.06** | **21920.33** |
| **13** | **S267V/S298L/L 328W** | **0.90** | **0.13** | **0.00** | **0.00** | **7.21** | **73.61** | **3.92** | **2081.88** | **5547.67** |
| **14** | **S239VN266L/S 298L/L328W** | **0.33** | **0.07** | **0.00** | **NA** | **5.02** | **39.07** | **-** | **-** | **-** |
| **15** | **A235W/V266L/ S298L/L328W** | **0.22** | **0.05** | **0.00** | **NA** | **4.67** | **70.06** | **-** | **-** | **-** |
| **16** | **P233FN266L/S 298L/L328W** | **0.29** | **0.16** | **0.00** | **NA** | **1.79** | **94.73** | **-** | **-** | **-** |
| **17** | **S267V/S298G/L 328W** | **0.19** | **0.03** | **0.00** | **NA** | **5.76** | **47.36** | **-** | **-** | **-** |
| **18** | **V234M/S267E/S 298L/L328W** | **0.53** | **0.10** | **NA** | **NA** | **5.13** | **-** | **-** | **-** | **-** |
| **19** | **V234M/S267E/S 298G/I332L** | **0.30** | **0.46** | **0.01** | **0.00** | **0.66** | **64.37** | **16.47** | **695.04** | **1852.11** |
| **#4** | **WT** | **12.58** | **5.25** | **2.51** | **8.97** | **2.40** | **2.09** | **0.28** | **1.40** | **1.00** |
| **20** | **V266L/S267E/H 268S/E269A/P2 71C** | **6.76** | **0.95** | **0.25** | **0.00** | **7.11** | **3.84** | **60.52** | **1651.90** | **1178.72** |
| **21** | **V266L/S267E/H 268S/E269V** | **4.15** | **2.13** | **0.17** | **0.00** | **1.95** | **12.85** | **99.90** | **2501.18** | **1784.72** |
| **22** | **V266L/S267E/H 268S/E269V/P2 71C** | **0.11** | **0.01** | **0.00** | **NA** | **12.64** | **6.97** | **-** | **-** | **-** |
| **23** | **V266L/S267E/H 268S/E269G/P2 71T** | **1.23** | **0.24** | **0.03** | **0.00** | **5.20** | **9.34** | **32.69** | **1588.00** | **1133.12** |
| **24** | **V266L/S267D/H 268D/E269D/P2 71Q** | **1.88** | **0.49** | **0.12** | **0.00** | **3.80** | **4.07** | **121.76** | **1882.93** | **1343.57** |
| **25** | **V266L/S267E/E 269K/P271G** | **1.22** | **0.42** | **0.14** | **0.00** | **2.94** | **2.92** | **214.29** | **1838.25** | **1311.69** |
| **26** | **V234Q/V266L/S 267D/H268D/E2 69D/P271Q** | **0.04** | **0.00** | **NA** | **NA** | **15.39** | **-** | **-** | **-** | **-** |
| **27** | **V234Q/V266L/S 267E/H268S/E2 69V** | **0.09** | **0.01** | **NA** | **NA** | **11.37** | **-** | **-** | **-** | **-** |
| **28** | **V266L/S267M/ H268E/P271Q** | **13.35** | **17.07** | **2.59** | **0.01** | **0.78** | **6.60** | **229.11** | **1182.52** | **843.79** |

| | | | | | | | | | | |
|---|---|---|---|---|---|---|---|---|---|---|
| # 1-4: Data from the experiments of Libraries IgG2_C01 (#1), IgG2_C02 (#2), Cmix (#3), C2 (#4). NA: In the sample of the column, the mutation of the row is not detected. | | | | | | | | | | |

### Example 9: The binding property of mutant antibodies to FcyR analyzed by Enzyme-linked immunosorbent assay (ELISA)

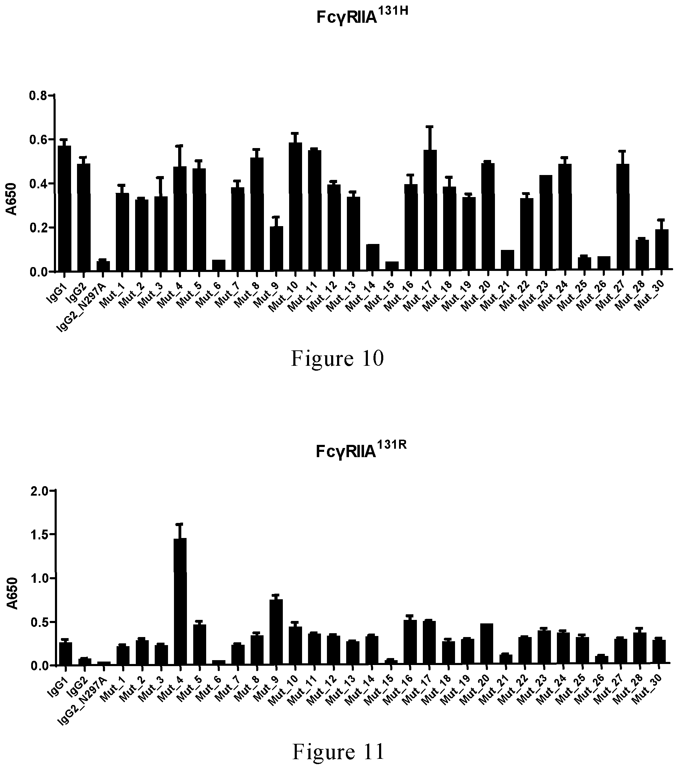

On the basis of the favored Fc single-point mutation and combined mutation screened above by NGS, new Fc mutation combinations are constructed. Anti-human OX40 antibodies containing these new Fc mutation combinations are expressed and the binding property of the mutant antibody to FcyR are analyzed by Enzyme-linked immunosorbent assay (ELISA). FIGS. 9-14 show the binding affinity of different Fc mutants to FcyRI, FcγRIIA^{131H}, FcγRIIA^{131R}, FcyRIIB, FcγRIIIA^{158F} and FcγRIIIA^{158V}. By designating the binding affinity of IgG1 to each FcyR to 1, the relative binding affinity of each mutant to the FcyR (the ELISA absorbance value of the binding of the mutant to the FcyR divided by the ELISA absorbance value of the binding of IgG1 to the FcyR) and the increased factor of the binding affinity of each mutant to FcyRIIB as compared with that of wild type IgG2 (the ELISA absorbance value of the binding of the mutant to FcyRIIB divided by the ELISA absorbance value of the binding of wild-type IgG2 to FcyRIIB) are calculated. As shown in Table 13, the increased factor of the binding affinity of each mutant to FcyRIIB as compared with that of wild-type IgG2 is 4.23 times to 25.55 times. In addition, the ratio of the binding affinity of each mutant to FcyRIIB to the binding affinity of said mutant to FcγRIIA^{131R} (RIIB/RIIAR) is calculated, and the results (Table 13) show that the RIIB/RIIAR ratio of these mutants is higher than that of wild-type IgG2 (wild-type IgG2: 0.34; mutants: 0.46-3.67).

**Table 13. Binding of different IgG2 antibody Fc mutants to FcyR.**

| | **Absorbance value** | | | | | | **Relative binding affinity** | | | | | | | |
|---|---|---|---|---|---|---|---|---|---|---|---|---|---|---|
| | **RI** | **RII AH** | **RII AR** | **RIIB** | **RII IAF** | **RII I A V** | **RI/ G1** | **RII** AH/ **G1** | **RIIA R/G1** | **RIIB/ G1** | **Increa sed fa ctor o f RII B bin ding comp ared with wild-t ype IgG2** | **RIIIA F/Gl** | **RIII A V/ G1** | **RIIB/ RIIA** R |
| IgG1 | 2.66 | 0.57 | 0.26 | **0.76** | 0.19 | 0.96 | | | | | | | | |
| IgG2 | 0.08 | 0.49 | 0.08 | **0.08** | 0.06 | 0.05 | 0.03 | 0.85 | 0.29 | **0.10** | **1.00** | 0.30 | 0.06 | **0.34** |
| IgG2 N297 A | 0.08 | 0.04 | 0.04 | **0.07** | 0.06 | 0.06 | 0.03 | 0.08 | 0.16 | **0.09** | **0.93** | 0.31 | 0.06 | **0.57** |
| Mut 1 | 0.17 | 0.35 | 0.22 | **0.64** | 0.07 | 0.09 | 0.06 | 0.62 | 0.83 | **0.85** | **8.51** | 0.38 | 0.09 | **1.02** |
| Mut 2 | 0.14 | 0.32 | 0.29 | **1.00** | 0.07 | 0.06 | 0.05 | 0.56 | 1.09 | **1.33** | **13.38** | 0.38 | 0.06 | **1.21** |
| Mut 3 | 0.18 | 0.34 | 0.23 | **0.94** | 0.08 | 0.07 | 0.07 | 0.60 | 0.87 | **1.25** | **12.57** | 0.40 | 0.08 | **1.44** |
| Mut 4 | 0.25 | 0.48 | 1.44 | **1.92** | 0.06 | 0.05 | 0.10 | 0.84 | 5.51 | **2.54** | **25.55** | 0.32 | 0.06 | **0.46** |
| Mut 5 | 0.38 | 0.46 | 0.46 | **1.18** | 0.06 | 0.06 | 0.14 | 0.81 | 1.77 | **1.56** | **15.69** | 0.31 | 0.07 | **0.88** |
| Mut 6 | 0.09 | 0.05 | 0.05 | **0.50** | 0.06 | 0.06 | 0.03 | 0.08 | 0.19 | **0.66** | **6.63** | 0.30 | 0.06 | **3.52** |
| Mut 7 | 0.13 | 0.38 | 0.23 | **0.58** | 0.07 | 0.06 | 0.05 | 0.67 | 0.87 | **0.76** | **7.70** | 0.38 | 0.07 | **0.88** |
| Mut 8 | 0.14 | 0.52 | 0.34 | **1.07** | 0.08 | 0.07 | 0.05 | 0.90 | 1.30 | **1.42** | **14.30** | 0.44 | 0.07 | **1.09** |
| Mut 9 | 0.12 | 0.20 | 0.74 | **1.20** | 0.07 | 0.07 | 0.05 | 0.34 | 2.83 | **1.59** | **15.99** | 0.38 | 0.08 | **0.56** |
| Mut_1 0 | 0.18 | 0.58 | 0.43 | **1.05** | 0.09 | 0.09 | 0.07 | 1.02 | 1.64 | **1.40** | **14.06** | 0.48 | 0.09 | **0.85** |
| Mut_1 1 | 0.13 | 0.55 | 0.36 | **1.01** | 0.08 | 0.08 | 0.05 | 0.96 | 1.36 | **1.33** | **13.40** | 0.41 | 0.08 | **0.98** |
| Mut_1 2 | 0.21 | 0.39 | 0.33 | **0.94** | 0.06 | 0.06 | 0.08 | 0.68 | 1.26 | **1.25** | **12.57** | 0.32 | 0.06 | **0.99** |
| Mut_1 3 | 0.18 | 0.33 | 0.26 | **0.73** | 0.07 | 0.08 | 0.07 | 0.58 | 1.01 | **0.97** | **9.75** | 0.37 | 0.09 | **0.96** |
| Mut_1 4 | 0.12 | 0.12 | 0.32 | **0.70** | 0.06 | 0.05 | 0.04 | 0.21 | 1.22 | **0.93** | **9.36** | 0.31 | 0.06 | **0.76** |
| Mut_1 5 | 0.08 | 0.04 | 0.04 | **0.47** | 0.06 | 0.05 | 0.03 | 0.07 | 0.17 | **0.62** | **6.21** | 0.31 | 0.06 | 3.67 |
| Mut_1 6 | 0.10 | 0.39 | 0.50 | **1.09** | 0.06 | 0.06 | 0.04 | 0.68 | 1.92 | **1.44** | **14.51** | 0.32 | 0.06 | **0.75** |
| Mut_1 7 | 0.14 | 0.54 | 0.50 | **1.19** | 0.09 | 0.06 | 0.05 | 0.95 | 1.90 | **1.58** | **15.93** | 0.49 | 0.06 | **0.83** |
| Mut_1 8 | 0.14 | 0.38 | 0.26 | **0.78** | 0.09 | 0.08 | 0.05 | 0.67 | 1.00 | **1.04** | **10.43** | 0.47 | 0.09 | **1.03** |
| Mut_1 9 | 0.11 | 0.33 | 0.28 | **0.82** | 0.07 | 0.07 | 0.04 | 0.58 | 1.08 | **1.09** | **10.96** | 0.38 | 0.07 | **1.00** |
| Mut_2 0 | 0.12 | 0.48 | 0.46 | **1.13** | 0.06 | 0.05 | 0.05 | 0.85 | 1.76 | **1.50** | **15.08** | 0.31 | 0.06 | **0.85** |
| Mut_2 1 | 0.16 | 0.09 | 0.11 | **0.32** | 0.06 | 0.06 | 0.06 | 0.16 | 0.41 | **0.42** | **4.23** | 0.31 | 0.06 | **1.03** |
| Mut_2 2 | 0.12 | 0.33 | 0.31 | **0.93** | 0.06 | 0.07 | 0.05 | 0.57 | 1.17 | **1.22** | **12.33** | 0.33 | 0.07 | **1.05** |
| Mut_2 3 | 0.11 | 0.43 | 0.38 | **0.97** | 0.07 | 0.06 | 0.04 | 0.75 | 1.44 | **1.29** | **12.96** | 0.35 | 0.06 | **0.90** |
| Mut_2 4 | 0.12 | 0.48 | 0.35 | **1.00** | 0.08 | 0.08 | 0.05 | 0.84 | 1.35 | **1.32** | **13.33** | 0.42 | 0.08 | **0.98** |
| Mut_2 5 | 0.12 | 0.06 | 0.30 | **0.86** | 0.07 | 0.05 | 0.05 | 0.10 | 1.13 | **1.14** | **11.49** | 0.36 | 0.06 | **1.01** |
| Mut_2 6 | 0.19 | 0.06 | 0.08 | **0.55** | 0.08 | 0.09 | 0.07 | 0.11 | 0.32 | **0.72** | **7.28** | 0.41 | 0.09 | **2.24** |
| Mut_2 7 | 0.18 | 0.48 | 0.28 | **0.70** | 0.09 | 0.10 | 0.07 | 0.84 | 1.06 | **0.92** | **9.29** | 0.49 | 0.11 | **0.87** |
| Mut_2 8 | 0.13 | 0.14 | 0.35 | **0.88** | 0.08 | 0.08 | 0.05 | 0.24 | 1.35 | **1.17** | **11.76** | 0.44 | 0.08 | **0.87** |
| Mut_3 0 | 0.12 | 0.18 | 0.26 | **0.80** | 0.06 | 0.06 | 0.05 | 0.32 | 1.01 | **1.06** | **10.65** | 0.33 | 0.07 | 1.05 |

| | | | | | | | | | | | | | | |
|---|---|---|---|---|---|---|---|---|---|---|---|---|---|---|
| Note: RI, RIIAH, RIIAR, RIIB, RIIIAF, RIIIAV respectively represent FcyRI, FcγRIIA^{131H}, FcγRIIA^{131R}, FcyRIIB, FcγRIIIA^{158F}, FcγRIIIA^{158V}. | | | | | | | | | | | | | | |

### Example 10: Beneficial mutations support stronger immune activation activity

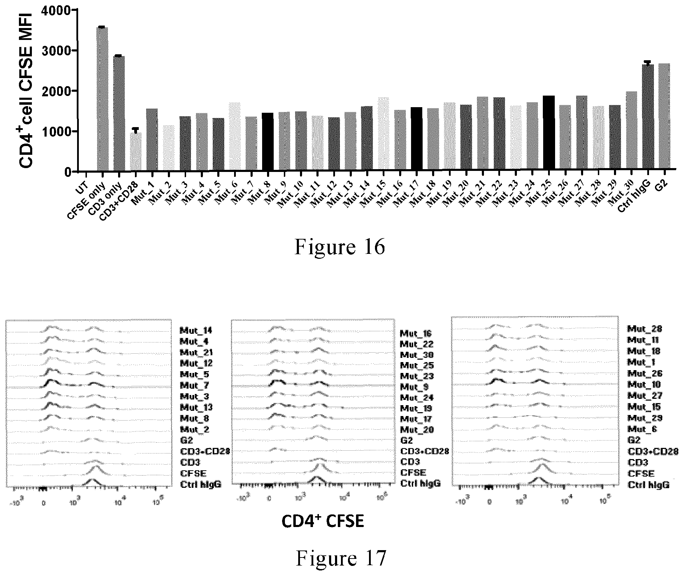

The immune activation activity of the anti-human OX40 antibody (Example 9) containing the beneficial mutation is analyzed in the FCyR/OX40 humanized mouse spleen cells. FIGS. 16 and 17 show the CFSE fluorescence intensity and the corresponding histogram of CD4⁺ cells when the antibody concentration is 1 µg/mL, where low CFSE fluorescence intensity reflects high activity, and the activity of all the selected antibodies is better than that of the wild-type IgG2. The activity values of different antibody concentrations of the two experiments are analyzed together. The activity of the group with αCD3 antibody only was set as 0, while the activity of the positive control group with αCD3 antibody and αCD28 antibody was set as 1. The relative activity of each mutant antibody is calculated, and the average value is calculated. Table 14 shows that the activity of the selected 30 mutant antibodies is superior to that of the wild type IgG2, and the activity is increased to 9.17 to 16.51 times of the wild type IgG2.

**Table 14. Immunostimulatory activity analysis of anti-human OX40 antibodies containing different Fc mutations**

| | **Experiment 1** | | | | | | | **Experiment 2** | | | | | | |
|---|---|---|---|---|---|---|---|---|---|---|---|---|---|---|
| | **CFSE fluorescen ce intensity** | | | **Relative activity** | | | **Ave rag e r elat ive acti vity** | **CFSE fluor escence inte nsity** | | **Relative ac tivity** | | **Ave rage rel ativ e ac tivit y** | **Aver age r elativ e acti vity of tw o exp erime nts** | **Averag e incre ased fa ctor of the ac tivity o ver wil d-type IgG2 of two ex perime nts** |
| | 1 µ g/m 1 | 0.1µ g/m 1 | 0.01 µg/ ml | 1 µ g/m 1 | 0.1µ g/m 1 | 0.01 µg/ ml | | 1µg /ml | 0.1µ g/ml | 1 µ g/ml | 0.1µ g/ml | | | |
| CD3+C D28 | 509 | 473 | 491 | 0.97 | 1.03 | 1.00 | 1.00 | 965 | 965 | 1.00 | 1.00 | 1.00 | 1.00 | |
| Mut_1 | 799 | 786 | 894 | 0.44 | 0.47 | 0.27 | 0.39 | 1552 | 2062 | 0.69 | 0.42 | 0.56 | 0.46 | 10.48 |
| Mut 2 | 677 | 654 | 685 | 0.66 | 0.71 | 0.65 | 0.67 | 1149 | 1548 | 0.90 | 0.69 | 0.80 | 0.72 | 16.51 |
| Mut 3 | 686 | 568 | 761 | 0.65 | 0.86 | 0.51 | 0.67 | 1365 | 1769 | 0.79 | 0.58 | 0.68 | 0.68 | 15.47 |
| Mut 4 | 769 | 616 | 695 | 0.50 | 0.77 | 0.63 | 0.63 | 1439 | 1702 | 0.75 | 0.61 | 0.68 | 0.65 | 14.91 |
| Mut 5 | 791 | 695 | 859 | 0.46 | 0.63 | 0.33 | 0.47 | 1309 | 1660 | 0.82 | 0.63 | 0.73 | 0.58 | 13.14 |
| Mut 6 | 706 | 689 | 766 | 0.61 | 0.64 | 0.50 | 0.59 | 1698 | 1986 | 0.61 | 0.46 | 0.54 | 0.57 | 12.94 |
| Mut 7 | 741 | 755 | 850 | 0.55 | 0.52 | 0.35 | 0.47 | 1347 | 1819 | 0.80 | 0.55 | 0.67 | 0.55 | 12.66 |
| Mut 8 | 799 | 719 | 844 | 0.44 | 0.59 | 0.36 | 0.46 | 1436 | 1813 | 0.75 | 0.55 | 0.65 | 0.54 | 12.32 |
| Mut 9 | 744 | 742 | 849 | 0.54 | 0.55 | 0.35 | 0.48 | 1454 | 1909 | 0.74 | 0.50 | 0.62 | 0.54 | 12.27 |
| Mut_10 | 765 | 743 | 788 | 0.50 | 0.54 | 0.46 | 0.50 | 1465 | 2048 | 0.74 | 0.43 | 0.58 | 0.54 | 12.23 |
| Mut_11 | 783 | 831 | 782 | 0.47 | 0.39 | 0.47 | 0.44 | 1359 | 1868 | 0.79 | 0.52 | 0.66 | 0.53 | 12.10 |
| Mut_12 | 762 | 689 | 906 | 0.51 | 0.64 | 0.25 | 0.47 | 1319 | 2085 | 0.81 | 0.41 | 0.61 | 0.52 | 11.99 |
| Mut_13 | 802 | 728 | 899 | 0.44 | 0.57 | 0.26 | 0.42 | 1446 | 1865 | 0.75 | 0.53 | 0.64 | 0.51 | 11.62 |
| Mut_14 | 793 | 775 | 839 | 0.45 | 0.49 | 0.37 | 0.44 | 1581 | 1899 | 0.68 | 0.51 | 0.59 | 0.50 | 11.40 |
| Mut_15 | 691 | 707 | 837 | 0.64 | 0.61 | 0.37 | 0.54 | 1826 | 2253 | 0.55 | 0.32 | 0.43 | 0.50 | 11.37 |
| Mut_16 | 783 | 770 | 853 | 0.47 | 0.50 | 0.35 | 0.44 | 1487 | 2158 | 0.72 | 0.37 | 0.55 | 0.48 | 11.01 |
| Mut_17 | 775 | 788 | 841 | 0.49 | 0.46 | 0.37 | 0.44 | 1557 | 2120 | 0.69 | 0.39 | 0.54 | 0.48 | 10.95 |
| Mut_18 | 776 | 828 | 838 | 0.48 | 0.39 | 0.37 | 0.42 | 1535 | 2135 | 0.70 | 0.38 | 0.54 | 0.47 | 10.65 |
| Mut_19 | 824 | 735 | 880 | 0.40 | 0.56 | 0.30 | 0.42 | 1672 | 2089 | 0.63 | 0.41 | 0.52 | 0.46 | 10.46 |
| Mut_20 | 774 | 844 | 828 | 0.49 | 0.36 | 0.39 | 0.41 | 1610 | 2161 | 0.66 | 0.37 | 0.51 | 0.45 | 10.37 |
| Mut_21 | 782 | 711 | 920 | 0.47 | 0.60 | 0.22 | 0.43 | 1814 | 2194 | 0.55 | 0.35 | 0.45 | 0.44 | 10.08 |
| Mut_22 | NA | NA | NA | - | - | - | - | 1791 | 2290 | 0.56 | 0.30 | 0.43 | 0.43 | 9.90 |
| Mut_23 | 843 | 777 | 898 | 0.36 | 0.48 | 0.26 | 0.37 | 1600 | 2161 | 0.67 | 0.37 | 0.52 | 0.43 | 9.80 |
| Mut_24 | 816 | 812 | 912 | 0.41 | 0.42 | 0.24 | 0.36 | 1654 | 2052 | 0.64 | 0.43 | 0.53 | 0.43 | 9.75 |
| Mut_25 | 779 | 778 | 895 | 0.48 | 0.48 | 0.27 | 0.41 | 1827 | 2209 | 0.55 | 0.34 | 0.45 | 0.42 | 9.69 |
| Mut_26 | 753 | 806 | 938 | 0.53 | 0.43 | 0.19 | 0.38 | 1591 | 2368 | 0.67 | 0.26 | 0.47 | 0.42 | 9.50 |
| Mut_27 | 834 | 807 | 849 | 0.38 | 0.43 | 0.35 | 0.39 | 1827 | 2214 | 0.55 | 0.34 | 0.44 | 0.41 | 9.36 |
| Mut_28 | 862 | 882 | 829 | 0.33 | 0.29 | 0.39 | 0.34 | 1566 | 2230 | 0.68 | 0.33 | 0.51 | 0.41 | 9.26 |
| Mut_29 | NA | NA | NA | - | - | - | - | 1581 | 2611 | 0.68 | 0.13 | 0.40 | 0.40 | 9.23 |
| Mut_30 | NA | NA | NA | - | - | - | - | 1925 | 2277 | 0.49 | 0.31 | 0.40 | 0.40 | 9.17 |
| WTIgG 2 | 106 0 | 101 6 | 100 2 | -0.0 3 | 0.05 | 0.08 | 0.03 | 2610 | 2885 | 0.13 | -0.0 1 | 0.06 | 0.04 | 1.00 |
| Ctrl hI gG | 107 1 | 108 3 | 101 8 | -0.0 5 | -0.0 7 | 0.05 | -0.0 2 | 2587 | 2730 | 0.15 | 0.07 | 0.11 | 0.03 | 0.65 |
| CD3 o nly | 105 1 | 103 7 | 104 4 | -0.0 1 | 0.01 | 0.00 | 0.00 | 2863 | 2863 | 0.00 | 0.00 | 0.00 | 0.00 | 0.00 |

| | | | | | | | | | | | | | | |
|---|---|---|---|---|---|---|---|---|---|---|---|---|---|---|
| NA: Mutant antibody of the row was not subjected to the experiment of the column. | | | | | | | | | | | | | | |

### Example 11: The effect of the known mutation on the binding ability of IgG2 to FcγR cannot be directly predicted according to the effect of the mutation on the binding ability of IgG1 to FcγR.

The affinity of human IgG1 to FcyRIIB can be increased by G236D, S239D (Seung Y Chu et al. Molecular Immunology 45 (2008) 3926-3933) and S298A mutation (US20090042291A1). However, in the results of NGS analysis of the present invention, the proportion of the IgG2 variant comprising G236D or S298A after sorting is not increased, but decreased (Table 10); the proportion of the human IgG2 variant containing S239D before sorting is 0.026%, and is not detected after sorting. These results demonstrate that G236D, S239D, and S298A mutations do not increase the FcyRIIB binding capacity of human IgG2, see Table 10.

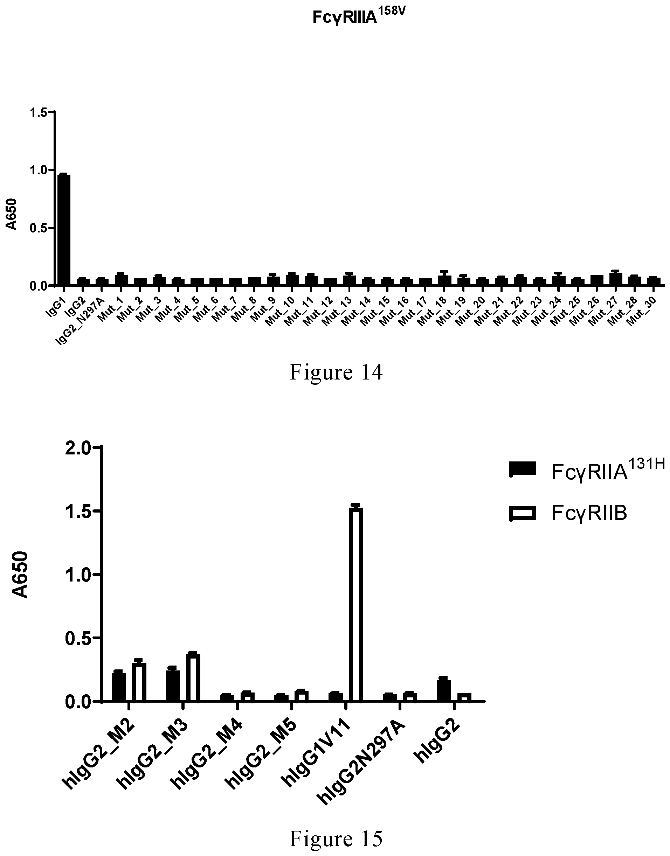

In addition, we also introduce into IgG2 the V11 mutations (G237D/P238D/H268D/P271G/A330R) capable of significantly improving the binding affinity of IgG1 to FcyRIIB (F Mimoto et al., Protein Engineering, Design & Selection vol. 26 no. 10 pp. 589-598, 2013) to obtain IgG2_M2(H268D/P271G), IgG2_M3(H268D/P271G/A330R), IgG2_M4 (G236D/P238D/H268D/P271G) and IgG2_M5(G236D/P238D/H268D/P271G/A330R), wherein the mutations of M5 in IgG2 corresponds to the mutations of IgG1 V11. ELISA results of FIG. 15 show that these mutants do not provide IgG2 with the same binding affinity to FcyRIIB as IgG1 V11.

Therefore, the effect of the known mutation on the binding ability of IgG2 to FcyR cannot be directly predicted according to the effect of the mutation on the binding ability of IgG1 to FcγR.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A mutant Fc polypeptide fragment, wherein the mutant Fc polypeptide fragment has the following features:
(i) said mutant Fc polypeptide fragment has a mutation with respect to a corresponding wild-type Fc fragment before mutation; and
(ii) the affinity of the mutant Fc region to FcyRIIB is increased compared to the wild-type Fc region; and the wild-type Fc is the Fc of wild-type IgG2.

2. A mutant immunoglobulin Fc region, wherein the mutant immunoglobulin Fc region comprises the mutant Fc polypeptide fragment according to claim 1.

3. An antibody, wherein the antibody comprises the mutant Fc polypeptide fragment according to claim 1 or the mutant immunoglobulin Fc region according to claim 2.

4. A fusion protein, wherein the fusion protein comprises the mutant Fc polypeptide fragment according to claim 1, the mutant immunoglobulin Fc region according to claim 2, or the antibody according to claim 3.

5. An isolated polynucleotide, wherein the polynucleotide encodes the mutant Fc polypeptide fragment according to claim 1, the mutant immunoglobulin Fc region according to claim 2, the antibody according to claim 3, or the recombinant protein according to claim 4.

6. A vector, wherein the vector comprises the isolated polynucleotide according to claim 5.

7. A host cell, wherein the host cell contains the vector according to claim 6, or having the polynucleotide according to claim 5 integrated into the genome of the cell;
or, the host cell expresses the mutant Fc polypeptide fragment according to claim 1, the mutant immunoglobulin Fc region according to claim 2, the antibody according to claim 3, or the recombinant protein according to claim 4.

8. A pharmaceutical composition, wherein the pharmaceutical composition comprises:
(a) the mutant Fc polypeptide fragment according to claim 1, the mutant immunoglobulin Fc region according to claim 2, the antibody according to claim 3, or the recombinant protein according to claim 4; and
(b) a pharmaceutically acceptable carrier.

9. A method of producing the mutant Fc polypeptide fragment according to claim 1, the mutant immunoglobulin Fc region according to claim 2, the antibody according to claim 3, or the recombinant protein according to claim 4, wherein the method comprises the steps of:
(i) culturing the host cell according to claim 7 under suitable conditions to obtain a culture comprising said mutant Fc polypeptide fragment, said mutant immunoglobulin Fc region, said antibody, or said recombinant protein; and
(ii) purifying and/or separating said mutant Fc polypeptide fragment, said mutant immunoglobulin Fc region, said antibody, or said recombinant protein from the culture obtained in step (i).

10. Use of the mutant Fc polypeptide fragment according to claim 1, the mutant immunoglobulin Fc region according to claim 2, the antibody according to claim 3, the recombinant protein according to claim 4, the polynucleotide according to claim 5, the vector according to claim 6 and/or the host cell according to claim 7, in the manufacture of a pharmaceutical composition for tumor immunotherapy, reducing inflammation and/or reducing autoimmune symptoms.
